# FASCICULE DE BREVET EUROPEEN

(11) **EP 2 193 204 B1**
(45) Date de publication et mention de la délivrance du brevet: **31.08.2011**
(21) Numéro de dépôt: 08839001.8
(22) Date de dépôt: 25.09.2008
(51) Int. Cl.: C12N 15/88, A61K 48/00, A61K 47/24, A61K 31/664, A61K 45/06, C07F 9/22, A61K 31/7084

(54) **COMPOSITIONS COMPRENANT DES LIPOPHOSPHORAMIDES ET LEURS UTILISATIONS EN THERAPIE GENIQUE**
LIPOPHOSPHORAMIDE ENTHALTENDE ZUBEREITUNGEN UND DEREN VERWENDUNGEN IN DER GENTHERAPIE
COMPOSITIONS COMPRISING LIPOPHOSPHORAMIDES AND THEIR USES IN GENE THERAPY

(30) Priorité: 28.09.2007 FR 0757955
(43) Date de publication de la demande: 09.06.2010
(73) Titulaire: Universite de Bretagne Occidentale, 29238 Brest Cedex 3 (FR); Centre National de la Recherche Scientifique, 75794 Paris Cedex 16 (FR)
(72) Inventeur: CLEMENT, Jean-Claude, 21300 Kranidi (GR); PICHON, Harivony, F-45550 Saint Denis de l'Hôtel (FR); MIDOUX, Patrick, F-45550 Saint Denis de l'Hôtel (FR); YAOUANC, Jean-Jacques, F-29280 Locmaria-Plouzane (FR); MEVEL, Mathieu, F-44000 Nantes (FR)
(74) Mandataire: Catherine, Alain
(86) Numéro de dépôt international: PCT/FR2008/051709
(87) Numéro de publication internationale: WO 2009/050372

(56) Documents cités:
- FR-A- 2 846 967
- MONTIER T ET AL: "KLN-5: a safe monocationic lipophosphoramide to transfect efficiently haematopoietic cell lines and human CD34<+> cells" BIOCHIMICA ET BIOPHYSICA ACTA. BIOMEMBRANES, AMSTERDAM, NL, vol. 1665, no. 1-2, 11 octobre 2004 (2004-10-11), pages 118-133, XP004592241 ISSN: 0005-2736
- MEVEL, MATHIEU ET AL: "Dicationic Lipophosphoramidates as DNA Carriers" BIOCONJUGATE CHEMISTRY , 18(5), 1604-1611 CODEN: BCCHES; ISSN: 1043-1802, 2007, XP002473043
- MILLER A D: "CATIONIC LIPOSOMES FOR GENE THERAPY" ANGEWANDTE CHEMIE. INTERNATIONAL EDITION, WILEY VCH VERLAG, WEINHEIM, DE, vol. 37, no. 13, 3 août 1998 (1998-08-03), pages 1768-1785, XP000772935 ISSN: 1433-7851 cité dans la demande
- MEVEL, MATHIEU ET AL: "Synthesis and transfection activity of new cationic phosphoramidate lipids: high efficiency of an imidazolium derivative" CHEMBIOCHEM , 9(9), 1462-1471 CODEN: CBCHFX; ISSN: 1439-4227, 2008, XP002541564
- MEVEL, MATHIEU ET AL: "Novel neutral imidazole-lipophosphoramides for transfection assays" CHEMICAL COMMUNICATIONS (CAMBRIDGE, UNITED KINGDOM) , (27), 3124-3126 CODEN: CHCOFS; ISSN: 1359-7345, 2008, XP002541565

## Description

### DOMAINE DE L'INVENTION

La présente invention se rapporte au domaine des compositions utilisables comme vecteurs non viraux pour introduire des acides nucléiques d'intérêt dans une cellule hôte humaine ou de mammifère non humain.

### ART ANTERIEUR

Dans les années récentes, de nombreux auteurs se sont intéressés au développement de vecteurs non-viraux destinés à transporter un ADN d'intérêt à travers la membrane cellulaire et jusqu'au noyau cellulaire, notamment dans le cadre de la mise au point de procédés de thérapie génique.

Dans la revue de A.D. MILLER intitulée « Cationic liposomes for gene therapy » (Angewandte Chem. Int., Ed. Engl., 1998, Vol. 37 : 1768-1785), qui constitue une revue générale sur les lipides cationiques, on peut noter que la charge positive du cation est toujours portée par un atome d'azote.

Parmi les composés lipophiles utilisés dans l'état de la technique comme vecteurs non viraux, on peut citer les halogénures de 1,2 dioleyl-3 trimethylammonium deoxyglycerol, communément appelé DOTAP, le 1,2 dioleyl-3 trimethylammonium, communément appelé DOTMA, le dimethylammonium ethyloxycarbonylcholesterol, communément désigné DC-chol.

On a aussi décrit des phosphonolipides tels que ceux décrits par G. Le Bolc'h et al., (Tetrahedron Lett., 1995, 36, 6681) et V. Floch et al (Eur. J. Med. Chem., 1998, 33, 12.), des phosphonolipides sous la forme d'un sel du cation ammonium (V. Floch et al., Eur. J. Med. Chem., 1998/, Vol. 33 : 923-934) ou sous la forme d'un sel du cation phosphonium ou arsonium (E. Guénin et al., Angew.Chem Int. Ed., 2000, Vol. 39(3); V. Floch et al., J. Med. Chem., 2000, Vol. 43 (24) : 4617-4628).

En général, les vecteurs lipophiles cationiques non-viraux ont une capacité réduite de transfection d'ADN dans des cellules et possèdent des propriétés cytotoxiques vis-à-vis de ces cellules.

On connaît néanmoins dans l'état de la technique certains vecteurs lipophiles cationiques qui possèdent de bons rendements de transfection combinés à une cytotoxicité faible.

Afin de réduire la cytotoxicité de vecteurs lipophiles cationiques, on a décrit dans l'état de la technique leur utilisation en combinaison avec des « co-lipides » neutres tels que la dioleoylphosphatidylethanolamine (DOPE) (voire FR2846967) ou encore le cholestérol. Toutefois, l'utilisation de ces co-lipides, qui permet de réduire les propriétés cytotoxiques de certains vecteurs lipophiles cationiques, présente des inconvénients. Il est connu, par exemple, que le co-lipide DOPE entraîne une réduction de la capacité de la composition lipidique vecteur résultante à transfecter ou transformer une cellule hôte avec un acide nucléique d'intérêt, du fait que la DOPE induit l'agglomération des complexes vecteurs lipophiles/acides nucléiques avec les lipoprotéines du sang.

Il existe donc un besoin dans l'état de la technique pour de nouvelles compositions lipophiles utilisables comme vecteurs d'acides nucléiques d'intérêt, qui possèdent une grande capacité de transfection et une cytotoxicité faible.

Il existe en particulier un besoin pour la disponibilité au public de co-lipides améliorés utilisables avec les vecteurs lipophiles cationiques.

Il existe aussi un besoin, dans l'état de la technique, pour des vecteurs non-viraux transportant plus efficacement un acide nucléique à travers la membrane cellulaire, jusqu'au noyau cellulaire, afin d'obtenir des rendements de transfection supérieurs à ceux observés avec les vecteurs non-viraux connus.

### RESUME DE L'INVENTION

Il est tout d'abord fourni selon l'invention de nouveaux co-lipides utilisables en combinaison avec des composés lipophiles vecteurs d'acides nucléiques, pour la fabrication d'une composition vecteur d'acides nucléiques.

L'invention fournit aussi des compositions vecteurs d'acides nucléiques comprenant la combinaison (i) d'un vecteur lipophile cationique d'acides nucléiques et (ii) d'un co-lipide. Ces compositions vecteurs d'acides nucléiques se présentent, dans certains modes de réalisation, sous la forme de vésicules unilamellaires ou multilamellaires.

L'invention est également relative à des procédés pour introduire *in vitro* ou *in* vivo un acide nucléique d'intérêt dans des cellules hôtes, comprenant une étape de mise en contact desdites cellules hôtes avec une composition vecteur d'acides nucléiques telle que définie ci-dessus.

La présente invention concerne aussi des complexes entre un acide nucléique d'intérêt et une composition vecteur d'acides nucléiques telle que définie ci-dessus.

L'invention a aussi trait à de nouveaux composés lipophiles cationiques vecteurs d'acides nucléiques et à leurs utilisations.

### DESCRIPTION DES FIGURES

Les Figures 1 à 6 représentent différents schémas de synthèse des composés lipophiles de formules (I) et (XI).

La Figure 1 représente le schéma de synthèse des composés lipophosphoramidates possédant une tête polaire constituée d'un dérivé d'acide aminé. La Figure 2 représente le schéma de synthèse du lysinemethylester-lipophosphoramidate 4 à partir du composé **3d**. La Figure 3 représente le schéma de synthèse des composés 5 et 6. La Figure 4 représente le schéma de synthèse d'un lipophosphoramidate à noyau imidazole **7b** et à noyau imidazolium **8b**. La Figure 5 représente le schéma de synthèse du composé **8a**. La Figure 6 représente le schéma de synthèse du composé **9**.

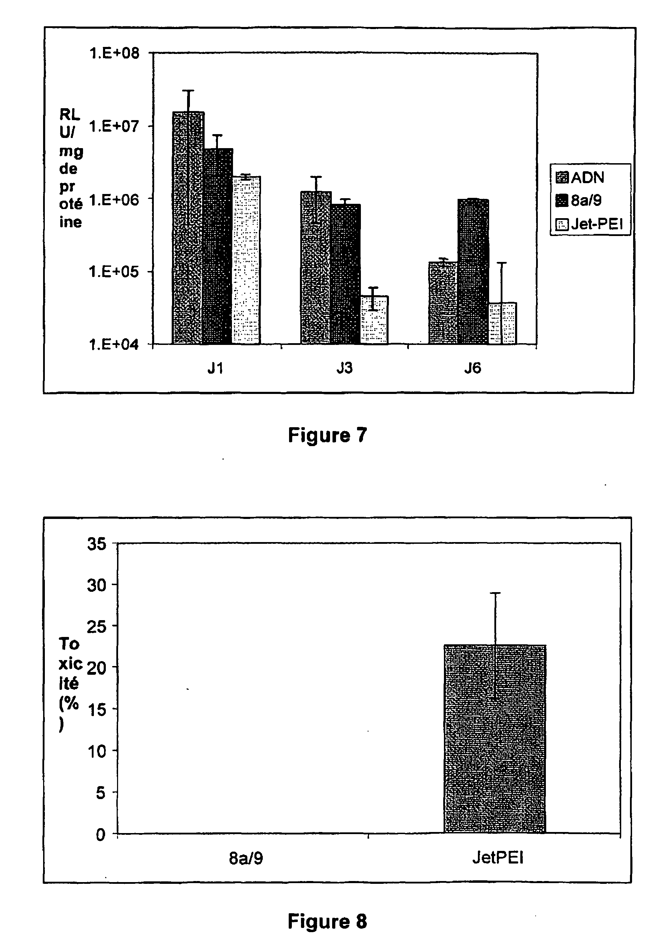

**Figure 7** **:** Cinétique d'expression du transgène dans les tendons de rats lésés. On injecte 20µg de pNFCMV-luc vectorisé ou non (40µL de volume final). L'activité luciférase est évaluée 1 ou 3 ou 6 jours après la transfection. Les résultats correspondent aux moyennes et écart-types de 3 expériences indépendantes effectuées en triple. L'activité luciférase du tendon collatéral non traité a été soustraite pour chaque rat traité. En abscisse : temps après la transfection, exprimé en jours ; barres de gauche à droite : ADN sans vecteur, ADN complexé avec une composition comprenant la combinaison des composés 8a et 9, ADN complexé avec le polymère Jet-PEI. Ordonnées : les résultats d'efficacité de transfection correspondant à l'activité luciférase retrouvée dans les échantillons de cellules transfectées en culture, exprimés en unités TRLU (« Total Relative Light Units »), comme décrit dans le partie « Matériels et Méthodes » des exemples.

**Figure 8** : Evaluation de la toxicité. Les ténocytes en culture ont été transfectés avec 8a/9 et JetPEI. Le test MTT a été effectué après 48h. Le pourcentage de toxicité a été établi par rapport à des cellules témoins non transfectées. En abscisse : le type d'échantillon. En ordonnées : le pourcentage de cytotoxicité.

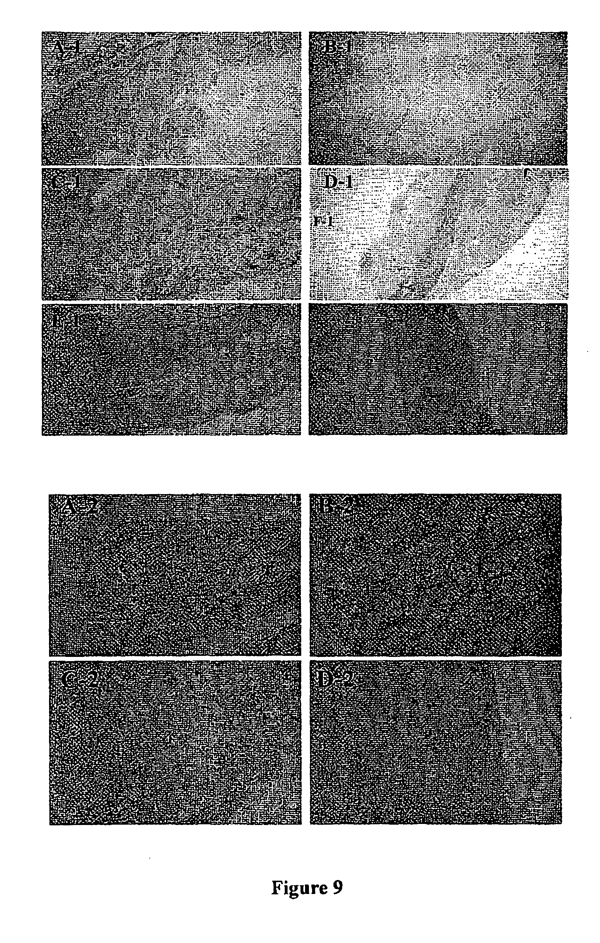

**Figure 9** :Analyses histologiques des tendons d'Achille
Les tendons ont été lésés chirurgicalement et transfectés (ou non) avec le plasmide pBlast hB-PDGF codant le facteur de croissance PDGF vectorisé avec 8a/9. Les tendons ont ensuite été prélevés à différents jours pour l'analyse histologique : coupes longitudinales (épaisseur 4µm) suivies d'une coloration HES.

| Grossissement 20X : | |
|---|---|
| 9A-1 et 9B-1 : | Tendon non lésé témoin |
| 9C-1 : | Tendon lésé, au 3ème jour, non traité |
| 9D-1 : | Tendon lésé, au 3ème jour, transfecté avec le cDNA du PDGF vectorisé avec 8a/9 |
| 9E-1 : | Tendon lésé, au 6ème jour, non traité |
| 9F-1 : | Tendon lésé, au 6ème jour, transfecté avec le pBlast hB-PDGF vectorisé avec 8a/9 |

| Grossissement 40X : | |
|---|---|
| 9A-2 : | Tendon non lésé témoin |
| 9B-2 : | Tendon lésé, au 3ème jour, non traité |
| 9C-2 : | Tendon lésé, au 6ème jour, non traité |
| 9D-2 : | Tendon lésé, au 6ème jour, transfecté avec le pBlast hB-PDGF vectorisé avec 8a/9 |

### DESCRIPTION DE L'INVENTION

Le demandeur a synthétisé de nouveaux composés lipophiles utilisables comme co-lipides dans des compositions de vecteurs non-viraux d'acides nucléiques.

Plus précisément, le demandeur a mis au point des composés lipophiles utilisables comme co-lipides, qui consistent en des composés de la famille des lipophosphoramidates, qui sont non-ionisés à un pH physiologique, et qui deviennent cationiques à un pH acide. Par « pH physiologique », on entend un pH allant de 7 à 7,6, et typiquement un pH de 7,4. Par « pH acide », on entend un pH inférieur à 7.

Un objet de l'invention consiste en l'utilisation d'un composé lipophile de formule (I) suivante : 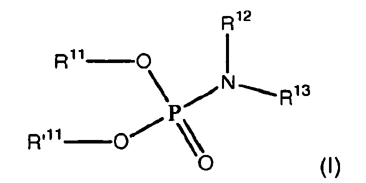
(i) R¹¹ et R'¹¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R¹² est un atome d'hydrogène ou une chaîne alkyle ayant de 1 à 4 atomes de carbone ;
(iii) R¹³ est choisi parmi :
   (iii-1) un groupe de formule 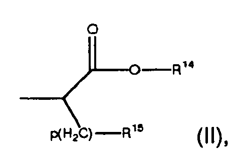 dans laquelle
      - R¹⁴ est un groupe alkyle ayant de 1 à 4 atomes de carbone ;
      - p est un entier égal à 1, 2, 3 ou 4 ; et
      - R¹⁵ est le groupe suivant : 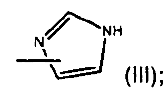 ou
   (iii-2) un groupe de formule -(CH₂)_{q}-R¹⁶, dans laquelle
      - q est un entier égal à 1, 2 , 3 ou 4 ;
      - R¹⁶ est le groupe suivant : 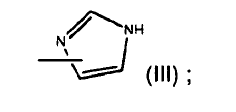
      comme co-lipide pour la fabrication d'une composition de vecteur non-viral d'acide nucléique.

On a montré selon l'invention qu'un composé lipophile de formule (I) ci-dessus possède la capacité d'accroître les propriétés de transfection de vecteurs lipophiles cationiques, y compris de vecteurs non-viraux lipophiles cationiques du type phosphoramide cationique.

On a aussi montré selon l'invention qu'un composé lipophile de formule (I) ci-dessus n'est pas cytotoxique. De plus, un composé lipophile de formule (I) ci-dessus, lorsqu'il est utilisé comme co-lipide en combinaison avec un composé vecteur lipophile cationique, permet la fabrication de compositions lipophiles vecteurs d'acides nucléiques qui possèdent une cytotoxicité réduite.

On a montré en particulier qu'avec les composés lipophiles de formule (I) utilisés comme co-lipides dans des compositions lipophiles vecteurs, la composition finale possède des propriétés de cytotoxicité très faibles, en comparaison des propriétés de cytotoxicité de compositions lipophiles vecteurs qui comprennent des co-lipides classiques comme la DOPE (L-α-dioleolyl-phosphatidylethanolamine).

Ainsi, les nouveaux composés lipophiles de formule (I) ci-dessus rendent possible la préparation de compositions vecteurs possédant les propriétés combinées (i) d'une haute capacité à transfecter des acides nucléiques d'intérêt dans des cellules hôtes et (ii) d'une cytotoxicité faible et même, dans certains modes de réalisation, d'une quasi-absence de propriétés cytotoxiques.

Dans un mode de réalisation préféré de l'utilisation d'un composé lipophile de formule (I) ci-dessus, ledit composé co-lipide est combiné à un composé lipophile cationique, apte à former un complexe avec un acide nucléique.

La présente invention a également pour objet une composition lipophile comprenant la combinaison de deux composés lipophiles, respectivement :
a) un premier composé lipophile cationique, apte à former un complexe avec un acide nucléique ; et
b) un second composé lipophile de formule (I) suivante : 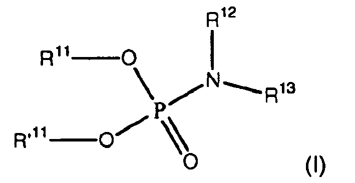
   (i) R¹¹ et R'¹¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
   (ii) R¹² est un atome d'hydrogène ou une chaîne alkyle ayant de 1 à 4 atomes de carbone ;
   (iii) R¹³ est choisi parmi :
      (iii-1) un groupe de formule 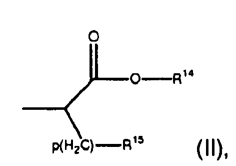 dans laquelle
         - R¹⁴ est un groupe alkyle ayant de 1 à 4 atomes de carbone ;
         - p est un entier égal à 1, 2, 3 ou 4 ; et
         - R¹⁵ est le groupe suivant : 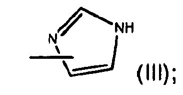 ou
      (iii-2) un groupe de formule -(CH₂)_{q}-R¹⁶, dans laquelle
         - q est un entier égal à 1, 2 , 3 ou 4 ;
         - R¹⁶ est le groupe suivant :

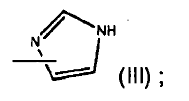

Par «alkyle», on entend selon l'invention un groupe hydrocarboné aliphatique, qui peut être linéaire ou ramifié. La chaîne alkyle peut être substituée, sur un ou plusieurs des atomes de carbone la constituant, par un ou plusieurs groupes choisis parmi les groupes méthyle, hydroxy, alcoxy et alkylthio. Préférentiellement, un atome de carbone de la chaîne hydrocarbonée comprend au maximum un seul substituant, mais ledit atome de carbone peut comprendre deux substituants. Dans la chaîne alkyle, tous les atomes de carbone peuvent comprendre au moins un substituant parmi les substituants cités ci-dessus.

Pour les groupes R¹¹ et R'¹¹, les chaînes alkyle, monoalcényle, polyalcényle, monoalcynyle et polyalcynyle possèdent de préférence de 14 à 20 atomes de carbones. Les groupes R¹¹ et R'¹¹ préférés englobent particulièrement les chaînes alkyle, monoalcényle, polyalcényle, monoalcynyle et polyalcynyle ayant 16 ou 18 atomes de carbone.

Dans certains modes de réalisation des composés lipophiles de formule (I), pour lesquels les groupes R¹¹ et/ou R'¹¹ représente(nt) une chaîne alkyle, ladite chaîne alkyle est substituée par au moins un groupe méthyle, par exemple par 2 à 8 groupes méthyle. Dans certains modes de réalisation, les groupes R¹¹ et/ou R'¹¹ représente(nt) un groupe phytanyle, c'est-à-dire une chaîne alkyle de 16 atomes de carbone avec quatre atomes de carbone monosubstituées par un groupe méthyle, respectivement les carbone en positions 3, 7, 11 et 15.

Par « monoalcényle », on entend selon l'invention un groupe alkyle contenant une double liaison carbone-carbone, qui peut être localisée à un endroit quelconque de la chaîne hydrocarbonée.

Par « polyalcényle », on entend selon l'invention un groupe alkyle contenant de deux à quatre double liaisons carbone-carbone dans la chaîne hydrocarbonée, qui peuvent être localisées à un endroit quelconque de la chaîne hydrocarbonée en positions « maloniques » relatives.

Par « monoalcynyle », on entend selon l'invention un groupe alkyle contenant une triple liaison carbone-carbone, qui peut être localisée à un endroit quelconque de la chaîne hydrocarbonée.

Par « polyalcynyle », on entend selon l'invention un groupe alkyle contenant de deux à quatre triple liaisons carbone-carbone dans la chaîne hydrocarbonée, qui peuvent être localisées à un endroit quelconque de la chaîne hydrocarbonée en positions « maloniques » relatives.

Selon l'invention, un complexe entre un composé lipophile cationique et un acide nucléique signifie que ledit acide nucléique est lié au composé lipophile cationique par des liaisons non-covalentes, du fait de la capacité des acides nucléiques, aussi bien ARNs qu'ADNs à s'associer sans liaison covalente à des substances chargées positivement.

Par « composé lipophile cationique »·, on entend selon l'invention un composé comprenant (i) au moins une chaîne hydrocarbonée lipophile et (ii) au moins un groupe chimique qui est chargé positivement à un pH physiologique, ledit composé étant apte à former un complexe avec un acide nucléique.

La composition lipophile définie ci-dessus constitue la composition lipophile vecteur non-viral selon l'invention. Cette composition lipophile forme un complexe avec des acides nucléiques d'intérêt. Comme précédemment indiqué, la composition lipophile ci-dessus possède d'excellentes propriétés de transfection de cellules hôtes avec des acides nucléiques, combinées à des propriétés cytotoxiques réduites.

Une première famille de composés de formule (I) consiste en la famille de composés de formule (I) pour laquelle le groupe R¹³ est un groupe de formule (II), qui est illustrée dans les exemples notamment par le composé référencé **3c**.

Selon un premier mode de réalisation préféré des composés de formule (I) avec le groupe R¹³ signifiant un groupe de formule (II), le groupe R¹² représente un atome d'hydrogène.

Selon un second mode de réalisation préféré des composés de formule (I) avec le groupe R¹³ signifiant un groupe de formule (II), le groupe R¹⁴ est un groupe méthyle.

Selon un troisième mode de réalisation préféré des composés de formule (I) avec le groupe R¹³ signifiant un groupe de formule (II), p est égal à 1, 3 ou 4.

Selon un quatrième mode de réalisation préféré des composés de formule (I) avec le groupe R¹³ signifiant un groupe de formule (II), le groupe R¹⁵ est choisi parmi les groupes suivants :

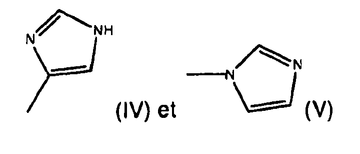

Une seconde famille de composés de formule (I) consiste en la famille de composés de formule (I) pour laquelle le groupe R¹³ est un groupe de formule -(CH₂)_{q}-R¹⁶, qui est illustrée dans les exemples notamment par les composés référencés **7b** et **9**.

Selon un premier mode de réalisation préféré des composés de formule (I) avec le groupe R¹³ signifiant un groupe de formule -(CH₂)_{q}-R¹⁶, le groupe R¹² représente un atome d'hydrogène.

Selon un second mode de réalisation préféré des composés de formule (I) avec le groupe R¹³ signifiant un groupe de formule -(CH₂)_{q}-R¹⁶, q est égal à 2 ou 3.

Selon un troisième mode de réalisation préféré des composés de formule (I) avec le groupe R¹³ signifiant un groupe de formule -(CH₂)_{q}-R¹⁶, le groupe R¹⁶ est choisi parmi les groupes suivants :

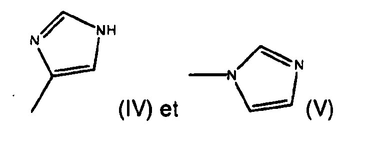

Selon un mode de réalisation préféré des composés de formule (I), les groupes R¹¹ et R'¹¹ sont, indépendamment l'un de l'autre, choisis parmi :
- le groupe tetradecyl,
- le groupe oleyl,
- le groupe phytanyl
- les groupes polyalcényl C_{18 :2} et C_{18 :3}, dans lequel le premier nombre représente le nombre d'atomes de carbone dans la chaîne alcényle et le second nombre représente le nombre de double liaisons dans la chaîne alcényle ; et
- le groupe monoalcényle C_{18 :1}, dans lequel le premier nombre représente le nombre d'atomes de carbone dans la chaîne alcényle et le second nombre représente le nombre de double liaisons dans la chaîne alcényle.

Selon un autre mode de réalisation préféré des composés de formule (I), les groupes R¹¹ et R'¹¹ sont identiques.

De manière tout à fait préférée, les groupes R¹¹ et R'¹¹ représentent chacun un groupe oleyl.

Les composés lipophiles de formule (I) selon l'invention consistent en des composés non-ionisés lorsqu'ils sont en solution à un pH neutre physiologique (pH de 7,6). En revanche, les composés lipophiles de formule (I) consistent en des composés ionisés cationiques lorsqu'ils sont en solution à un pH acide inférieur à 7, typiquement à un pH acide inférieur à 6.

Sans vouloir être lié par une quelconque théorie, le demandeur pense que les composés lipophiles de formule (I) possèdent de bonnes propriétés de fusion avec les lipides, et en particulier avec les lipides membranaires des cellules ou de certaines vésicules intracellulaires, y compris les endosomes. Le caractère cationique en milieu acide des composés lipophiles de formule (I) selon l'invention est de nature à favoriser la déstabilisation des vésicules endosomiques du cytoplasme cellulaire suivant une osmose, du fait qu'il est connu que les vésicules endosomiques se comportent comme des pompes à protons. Ainsi, après avoir été combinés avec des vecteurs lipophiles cationiques, les composés lipophiles de formule (I) favorisent, en tant que co-lipides, la cationisation des compositions lipophiles vecteurs d'acides nucléiques selon l'invention dans le cytoplasme cellulaire, et plus particulièrement dans les vésicules d'endosomes, ce qui favoriserait la délivrance des acides nucléiques dans le cytosol et permettrait d'expliquer leurs propriétés d'accroissement des rendements de transfection des cellules avec les acides nucléiques d'intérêt.

Dans une composition lipophile vecteur d'acides nucléiques selon l'invention, le composé lipophile cationique utilisé en combinaison avec le co-lipide de formule (1) peut être l'un quelconque des composés lipophiles cationiques connus, qui sont aptes à former un complexe avec un acide nucléique, ADN ou ARN. De manière générale, un composé lipophile cationique vecteur d'acide nucléique possède : (i) une parie lipophile, en général une ou plusieurs chaînes hydrocarbonées ayant de 10 à 24 atomes de carbones, saturée ou mono- ou poly-insaturée, (ii) une partie cationique qui est chargée positivement dans une solution à pH physiologique et (iii) un groupe de liaison (« linker ») qui relie la partie lipophile à la partie cationique, qui consiste en général en une liaison acyl ou en une liaison ether.

Par exemple, le composé lipophile cationique vecteur d'acide nucléique peut être choisi parmi le 1,2 dioleyl-3 trimethylammonium deoxyglycerol (DOTAP), le 1,2 dioleyl-3 trimethylammonium (DOTMA), le dimethylammonium ethyloxycarbonylcholesterol (DC-chol), le bromure de dimethyldioactadecyl ammonium (DDAB), le 1,2 dimyristoyl-3 trimethylammonium deoxyglycerol, 1,2 dipalmitoyl-3 trimethylammonium deoxyglycerol, le 1,2 dioleyl-3 trimethylammonium deoxyglycerol, le 1,2 distearoyl-3 trimethylammonium deoxyglycerol, le chlorure de N-[1-[2,3-bis(oleoyloxy)]propy-1]-N,N,N-trimethylammonium, le dioctadecylamidoglycylspermine (DOGS), le trifluoroacétate de 2,3-dioleoyloxy-N-(2(sperminecarboxamido)-ethyl)-N,N-dimethyl-1-propanaminium (DOSPA), le 1,2-dioleoyl-sn-glycero-3-ethylphosphocholine (DOEPC), le 1,2-dilauroyl-sn-glycero-3-ethylphosphocholine, le 1,2-dipalmitoyl-sn-glycero-3-ethylphosphocholine, le 1,2-distearoyl-sn-glycero-3-ethylphosphocholine, le 1,2-palimitoyl-oleoyl-sn-glycero-3-ethylphosphocholine, le chlorure de 1-[2-(9(Z)-octadecenoyloxy)ethyl]-2-(8(Z)-hepadecenyl 1-3-(2-hydroxyethyl)imidazolium .(DOTIM), le chlorure de 1-[2-tetradecanoyloxy)ethyl]-2-tridecyl-3-(2-hydroxyethyl) imidazolium (DPTIM), le chlorure 1-[2-tetradecanoyloxy)ethyl]-2-tridecyl-3-(2-hydroxyethyl) imidazolium, le bromure de 1,2-dioleoyl-3-dimethyl-hydroxyethyl ammonium (DOR1); le bromure de 1,2-dioleyloxypropyl-3-dimethyl-hydroxyethyl ammonium (DORIE); le bromure de 1,2-dioleyloxypropyl-3-dimethylhydroxypropyl ammonium (DORIE-HP); le bromure de 1,2-dioleyloxypropyl-3-dimethylhydroxybutyl ammonium (DORIE-HB); le bromure de 1,2-dioleyloxypropyl-3-dimethylhydroxypentyl ammonium (DORIE-HPe); le bromure de 1,2-dimyristyloxypropyl-3-dimethylhydroxylethyl ammonium (DMRIE); le bromure de 1,2-dipalmityloxypropyl-3-dimethyl-hydroxyethyl ammonium (DPRIE), le bromure de 1,2-disteryloxypropyl-3-dimethyl-hydroxyethyl ammonium (DSRIE) ; la L-histidine-(N,N-di-n-hexadecylamine)ethylamide (lipid 1); la L-histidine-(N,N-di-n-hexadecylamine,-N-methyl)ethylamide ; L-histidine-Cholesteryl-ethylamide, alanine-cholesteryl-ethylamide, et le bis(guanidinium)-tren-cholesterol (BGTC)

La totalité des composés lipophiles cationiques vecteurs d'acides nucléiques ci-dessus sont des produits accessibles dans le commerce ou bien leur synthèse est décrite dans la littérature technique.

Selon un mode de réalisation préféré d'une composition lipophile vecteur d'acides nucléiques selon l'invention, le composé lipophile cationique avec lequel est combiné le composé lipophile de formule (I), est un composé lipophile de formule (XI) suivante : 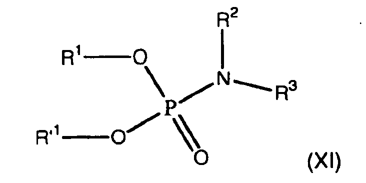 dans laquelle :
(i) R¹ et R'¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R² est un atome d'hydrogène ou une chaîne alkyle ayant de 1 à 4 atomes de carbone ;
(iii) R³ est choisi parmi :
   (iii-1) un groupe de formule 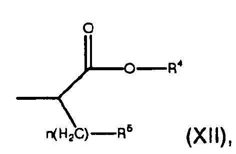 dans laquelle
      - R⁴ est un groupe alkyle ayant de 1 à 4 atomes de carbone ;
      - n est un entier égal à 1, 2, 3 ou 4 ; et
      - R⁵ est un groupe choisi parmi : 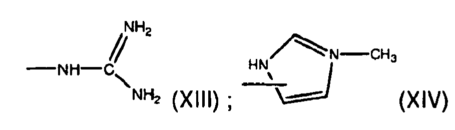 et -NH₃ ; ou
   (iii-2) un groupe de formule -(CH₂)ₒ-R⁶, dans laquelle
      - o est un entier égal à 1, 2, 3 ou 4 ;
      - R⁶ est un groupe choisi parmi : 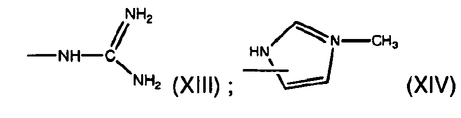
   et NH₃

On a montré selon l'invention que les composés lipophiles cationiques de formule (XI) définis ci-dessus constituent d'excellents composés lipophiles non-viraux vecteurs d'acides nucléiques. Les composés lipophiles cationiques de formule (XI) possèdent (i) une partie lipophile constituée de deux chaînes hydrocarbonées ayant de 10 à 24 atomes de carbone, (ii) une partie cationique qui est chargée positivement dans une solution à pH neutre physiologique, préférentiellement choisie parmi (ii-a) une chaîne latérale d'acide aminé chargée positivement à pH physiologique et (ii-b) un groupe imidazolium et (iii) un groupe de liaison (« linker ») de type phosphoramidate.

En particulier, on a montré selon l'invention que les composés de formule (XI) ci-dessus, et encore plus spécifiquement les composés de formule (XI) dans lesquels le groupe R³ est un groupe de formule (XII), avaient des propriétés cytotoxiques réduites, inférieures aux propriétés cytotoxiques des lipides cationiques vecteurs d'ADN couramment utilisés dans l'état de la technique, tels que le DOTAP et le DOTMA.

Dans les composés lipophiles cationiques de formule (XI) ci-dessus, le groupe R³ est protoné au pH physiologique, ce qui rend les composés de formule (XI) aptes à former des complexes avec des acides nucléiques.

En particulier, dans les composés lipophiles cationiques de formule (XI) pour lesquels le groupe R³ signifie un groupe de formule (XII), ledit groupe de formule (XII) constitue la partie d'un acide aminé comprenant le groupe carboxyle, qui est estérifié, ainsi que la chaîne latérale basique, qui est chargée positivement à pH physiologique.

Les composés lipophiles de formule (XI) pour lesquels le groupe R³ signifie un groupe de formule (XII) sont des composés nouveaux.

Une première famille de composés de formule (XI) consiste en la famille de composés de formule (XI) pour laquelle le groupe R³ signifie un groupe de formule (XII), qui est illustrée dans les exemples notamment par les composés référencés **3a**, **3b** et 4.

Selon un premier mode de réalisation préféré des composés de formule (XI) avec le groupe R³ signifiant un groupe de formule (XII), le groupe R² représente un atome d'hydrogène.

Selon un second mode de réalisation préféré des composés de formule (XI) avec le groupe R³ signifiant un groupe de formule (XII), le groupe R⁴ est un groupe méthyle.

Selon un troisième mode de réalisation préféré des composés de formule (XI) avec le groupe R³ signifiant un groupe de formule (XII), n est égal à 1, 3 ou 4.
Selon un quatrième mode de réalisation préféré des composés de formule (XI) avec le groupe R³ signifiant un groupe de formule (XII), le groupe R⁵ est le groupe suivant :

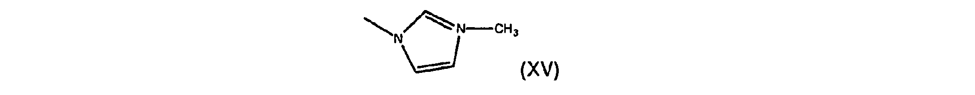

Une seconde famille de composés de formule (XI) consiste en la famille de composés de formule (XI) pour laquelle le groupe R³ est un groupe de formule -(CH₂)ₒ-R⁶, qui est illustrée dans les exemples notamment par les composés référencés **6a**, **6b**, **8a** et **8b**.

Selon un premier mode de réalisation préféré des composés de formule (XI) avec le groupe R³ signifiant un groupe de formule -(CH₂)ₒ-R⁶, le groupe R² représente un atome d'hydrogène.

Selon un second mode de réalisation préféré des composés de formule (XI) avec le groupe R³ signifiant un groupe de formule -(CH₂)ₒ-R⁶, o est égal à 2 ou 3.

Selon un troisième mode de réalisation préféré des composés de formule (XI) avec le groupe R³ signifiant un groupe de formule -(CH₂)ₒ-R⁶, le groupe R⁶ est le groupe suivant :

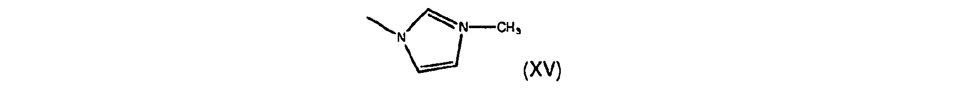

Selon un mode de réalisation préféré des composés de formule (XI), les groupes R¹ et R'¹ sont, indépendamment l'un de l'autre, choisis parmi :
- le groupe tetradecyl,
- le groupe oleyl,
- le groupe phytanyle,
- les groupes polyalcényl C_{18 :2} et C_{18 :3}, dans lequel le premier nombre représente le nombre d'atomes de carbone dans la chaîne alcényle et le second nombre représente le nombre de double liaisons dans la chaîne alcényle et;
- le groupe monoalcényle C_{18 :1}, dans lequel le premier nombre représente le nombre d'atomes de carbone dans la chaîne alcényle et le second nombre représente le nombre de double liaisons dans la chaîne alcényle.

Selon un autre mode de réalisation préféré des composés de formule (XI), les groupes R¹ et R'¹ sont identiques.

De manière tout à fait préférée, les groupes R¹ et R'¹ représentent chacun un groupe oleyl.

Dans un mode de réalisation préféré d'une composition lipophile vecteur d'acides nucléiques selon l'invention, le premier composé lipophile cationique de formule (XI) est choisi parmi les composés lipophiles suivants :
- le dioleoyl-N- phosphoramidate de l'ester méthylique de l'arginine référencé **3a**,
- le dioleoyl-N-phosphoramidate de l'ester méthylique de l'homoarginine référencé **3b**,
- le dioleoyl-N- phosphoramidate de l'ester méthylique de la lysine référencé **4**,
- le dioleoyl-N-phosphoramidate de 2-ethylguanidinium référencé **6a**,
- le dioleoyl-N-phosphoramidate de 4-butylguanidinium référencé **6b**,
- le dioleoyl-N-phosphoramidate de 2-ethyl(N-methyl)imidazolium référencé **8a**, et
- le dioleoyl-N-phosphoramidate de 3-propyl(N-methyl)imidazolium référencé **8b**.

Dans un autre mode de réalisation préféré d'une composition lipophile vecteur d'acides nucléiques selon l'invention, le second composé lipophile de formule (I) est choisi parmi les composés lipophiles suivants :
- le dioleoyl-N- phosphoramidate de l'ester méthylique de l'histidine référencé **3c**,
- le dioleoyl-N- phosphoramidate de 3-propylimidazole référencé **7b**, et
- le dioleoyl-N- phosphoramidate de l'histamine référencé **9**.

L'invention englobe toutes les combinaisons possibles des premiers et seconds composés lipophiles enseignés dans la présente description.

Une composition lipophile particulièrement préférée de l'invention consiste en la composition comprenant le premier composé lipophile **8a** et du second composé lipophile **9**.

Préférentiellement, une composition lipophile telle que définie précédemment dans la présente description se présente sous la forme de vésicules lipidiques, qui peuvent également être désignées liposomes.

Une composition lipophile selon l'invention est préférentiellement mise en oeuvre sous la forme de vésicules lipidiques, qui sont ensuite mises en contact avec un acide nucléique d'intérêt de manière à former un complexe entre ledit acide nucléique et les vésicules lipidiques ainsi préparées.

Un autre objet de l'invention consiste en des vésicules lipidiques essentiellement constituées d'une composition lipophile vecteur d'acides nucléiques telle que définie précédemment dans la description. Par « essentiellement », ont entend des vésicules lipidiques comprenant au moins 90% en poids d'une composition lipophile de l'invention, par rapport au poids total desdites vésicules lipidiques.

Les vésicules lipidiques sont préparées à partir d'un mélange de :
a) un premier composé lipophile cationique, apte à former un complexe avec un acide nucléique ; et
   et
b) un second composé lipophile de formule (I) suivante : 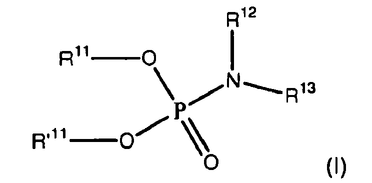
   (i) R¹¹ et R'¹¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
   (ii) R¹² est un atome d'hydrogène ou une chaîne alkyle ayant de 1 à 4 atomes de carbone ;
   (iii) R¹³ est choisi parmi :
      (iii-1) un groupe de formule 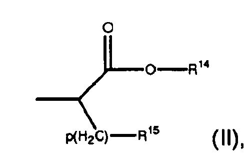 dans laquelle
         - R¹⁴ est un groupe alkyle ayant de 1 à 4 atomes de carbone ;
         - p est un entier égal à 1, 2, 3 ou 4 ; et
         - R¹⁵ est le groupe suivant : 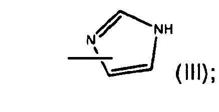 ou
      (iii-2) un groupe de formule -(CH₂)_{q}-R¹⁶, dans laquelle
         - q est un entier égal à 1, 2 , 3 ou 4 ;
         - R¹⁶ est le groupe suivant :

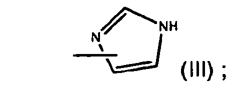

Comme déjà précédemment mentionné dans la présente description, le premier composé lipophile, qui consiste en un composé lipophile cationique, peut être l'un quelconque des composés lipophiles cationiques connus, qui sont aptes à former un complexe avec un acide nucléique, ADN ou ARN.

Pour le second composé lipophile de formule (I), les significations des groupes R¹¹, R'¹, R¹², R¹³, R¹⁴, R¹⁵ et R¹⁶ sont celles définies précédemment dans la présente description.

Font ainsi partie de l'invention des vésicules lipidiques comprenant un premier composé lipophile cationique tel que défini ci-dessus et un second composé lipophile consistant en un composé lipophile de formule (I) et qui est utilisé à titre de co-lipide.

Font également partie de l'invention des vésicules lipidiques constituées exclusivement d'une composition lipophile de l'invention telle que définie ci-dessus.

Dans une composition lipophile de l'invention, on utilise le premier composé lipophile cationique et le second composé lipophile neutre de formule (I) dans un rapport molaire composé cationique/composé neutre allant de 1/1 à 3/2

Dans le mode de réalisation particulier des vésicules lipidiques ci-dessus dans lequel lesdites vésicules comprennent moins de 100% en poids d'une composition lipophile de l'invention, lesdites vésicules comprennent jusqu'à 10% en poids d'une ou plusieurs autres substances additionnelles. Préférentiellement, la ou les substance(s) additionnelle(s) consistent en un ou plusieurs composés lipophiles additionnels, qui sont en général des composés lipophiles connus dans l'état de la technique, tels que la DOPE, la DOPC, ou le cholestérol, qui sont utilisés comme co-lipides.

De telles vésicules lipidiques comprennent au maximum quatre, et préférentiellement au maximum deux, composés lipophiles cationiques distincts selon l'invention. De même, de telles vésicules lipidiques comprennent au maximum quatre, et préférentiellement au maximum deux, composés lipophiles neutres distincts de l'invention.

Les vésicules lipidiques selon l'invention sont préparées selon toute technique connue de l'homme du métier, notamment les techniques de préparation de liposomes, y compris les techniques décrites dans les exemples. Par exemple, les vésicules lipidiques de l'invention peuvent être préparées par dissolution préalable du ou des composé(s) lipophile(s) dans un solvant organique, tel que de l'éthanol, puis par injection de la solution résultante dans un milieux aqueux, par exemple de l'eau distillée apyrogène ou une solution saline physiologiquement compatible, puis les vésicules lipidiques sont générées par traitement de la solution ainsi obtenue par des ultrasons, selon des techniques bien connues de l'homme du métier.

Lorsque les composés lipophiles sont dispersés dans de l'eau ou dans une solution aqueuse, ou encore dans toute solution hydrophile, lesdits composés lipophiles forment des membranes constituées de bi-couches lipidiques aussi désignées lamelles (« lamellae »). Les lamelles sont composées de deux mono-couches de composés lipophiles, avec leur surface hydrophobe l'une face à l'autre, et avec leur surface hydrophile en contact avec le milieu aqueux, c'est à dire à la fois avec le milieu aqueux de l'environnement extérieur et avec le milieu aqueux contenu dans l'espace interne des vésicules.

Selon certains modes de réalisation, les vésicules de l'invention consistent en des vésicules unilamellaires comprenant une seule bi-couche lipidique, qui ont généralement un diamètre allant de 100 à 200 nanomètres.

Selon certains autres modes de réalisation, les vésicules de l'invention consistent en des vésicules multilamellaires comprenant classiquement de 2 à quelques centaines de bi-couches lipidiques concentriques alternant avec des couches de milieu aqueux, qui ont généralement un diamètre allant de 100 à 200 nanomètres.

Les composés lipophiles en solution organique peuvent être conservées à long terme, par exemple à une température comprise entre 4 et 8°C. Une préparation extemporanée des vésicules est préférentiellement réalisée, au maximum quelques heures, par exemple au maximum 4 heures, avant leur mise en contact ou incubation avec un acide nucléique d'intérêt.

Avantageusement, l'acide nucléique d'intérêt qui doit être introduit dans une cellule hôte code une protéine ou un peptide. La protéine peut être n'importe quelle protéine utile pour la réalisation d'un procédé de thérapie génique, de préférence de thérapie génique somatique, et englobe, sans y être limitée, les cytokines, les protéines de structures, les hormones, les antigènes, les immunogènes, les récepteurs etc.

Selon un second mode de réalisation avantageux, l'acide nucléique d'intérêt code un polynucléotide sens ou antisens ou encore un ARN interférant qui s'hybride avec un acide nucléique cible codant une protéine dont l'inhibition de l'expression dans une cellule hôte est recherchée.

Selon un autre mode de réalisation avantageuse, l'acide nucléique d'intérêt consiste en un ARN messager codant une protéine d'intérêt.

Selon encore un autre mode de réalisation avantageux, l'acide nucléique d'intérêt consiste en un vecteur recombinant, de préférence un vecteur d'expression recombinant, dans lequel est inséré l'acide nucléique d'intérêt, dont la séquence codante est placée sous le contrôle de séquences régulatrices, notamment promoteur ou séquence activatrice (« enhancer »), nécessaires à l'expression dudit acide nucléique d'intérêt dans la cellule hôte transfectée.

Selon l'invention, l'acide nucléique d'intérêt, linéaire ou circulaire, simple brin, ou double brin, est tout d'abord complexé avec une composition lipophile selon l'invention, qui peut déjà se présenter sous formes de vésicules lipidiques, avant d'être introduit, sous la forme du complexe, dans la cellule hôte.

Toutefois, les complexes sont préférentiellement formés par incubation de l'acide nucléique avec les vésicules lipidiques définies ci-dessus.

Dans certains cas, les complexes peuvent être formés en incubant l'acide nucléique d'intérêt avec une composition lipophile de l'invention, les complexes ainsi formés étant ultérieurement utilisés pour la transfection des cellules hôtes. Selon une alternative, des vésicules lipidiques, unilamellaires ou multilamellaires, sont formées à partir des complexes acide nucléique/composé(s) lipophile(s) préalablement préparés, puis les vésicules sont utilisées pour transfecter les cellules hôtes.

L'invention a aussi pour objet l'utilisation d'une composition lipophile ou d'une vésicule lipidique tels que définis ci-dessus, pour introduire, *in vitro* ou *in vivo,* un acide nucléique dans une cellule hôte ou dans un organisme hôte.

L'invention concerne aussi un procédé pour introduire, *in vitro* ou *in vivo,* un acide nucléique dans une cellule hôte ou un organisme hôte, caractérisé en ce qu'il comprend les étapes suivantes :
a) mettre en contact ledit acide nucléique avec une composition lipophile ou avec une vésicule lipidique tels que définis ci-dessus, afin d'obtenir un complexe entre ledit acide nucléique, d'une part, et ladite composition ou ladite vésicule lipidique, d'autre part ;
b) incuber la cellule hôte avec le complexe formé à l'étape a), ou administrer, de préférence par injection, le complexe formé à l'étape a) à l'organisme hôte.

Préférentiellement, ladite cellule hôte est une cellule de mammifère non humain ou une cellule humaine

Préférentiellement, l'organisme hôte est un homme ou un mammifère non humain, bien que l'on ne puisse exclure l'application du procédé ci-dessus chez d'autres organismes supérieurs comme les plantes.

L'invention est également relative à un complexe formé entre un acide nucléique et une composition lipophile ou une vésicule lipidique tels que définis ci-dessus.

L'invention concerne aussi une composition comprenant un complexe formé entre un acide nucléique et une composition lipophile ou une vésicule lipidique tels que définis ci-dessus.

Comme cela a déjà été mentionné précédemment, les complexes formés entre un acide nucléique d'intérêt et un composé lipophile ou une vésicule lipidique de l'invention peuvent être administrés par toute méthode appropriée permettant leur introduction dans les cellules d'un homme ou d'un animal, tel que par injection dans les espaces interstitiels des tissus (coeur, muscle, peau, poumon, foie, intestins, etc.). De préférence, les complexes sont présentés sous la forme d'une composition contenant également un véhicule physiologiquement compatible.

Dans une forme de réalisation particulière d'administration des complexes selon l'invention, la composition contenant ces complexes se présente sous une forme adaptée à une administration par aérosol, par exemple pour inhalation.

Pour l'injection d'un complexe entre un composé lipophile ou d'une vésicule lipidique de l'invention et un acide nucléique d'intérêt, la quantité d'ADN, d'ARN ou d'ADN/ARN d'intérêt pour une dose injectable est avantageusement comprise entre 0,005 mg/kg et 50 mg/kg de poids de l'homme ou l'animal à traiter. De préférence, la quantité d'acide nucléique varie de 0,005 mg/kg à 20 mg/kg, et de manière tout à fait préférée de 0,05 mg/kg à 5 mg/kg.

Bien entendu, l'homme du métier est en mesure d'adapter la quantité d'acide nucléique dans une dose pour injection, en fonction notamment de la pathologie à traiter et du site d'injection.

La quantité d'acide nucléique pour une dose injectable est déterminée par l'homme du métier.

L'invention a également pour objet un procédé pour introduire *in vivo* un acide nucléique d'intérêt dans les cellules d'un organisme hôte, ledit procédé comprenant les étapes suivantes :
a) mettre en contact ledit acide nucléique avec une composition lipophile ou avec une vésicule lipidique tels que définis ci-dessus afin d'obtenir un complexe entre ledit acide nucléique, dune part, et ledit composé ou ladite vésicule lipidique, d'autre part ;
b) administrer les complexes formés à l'étape a) audit organisme hôte.

Comme déjà mentionné, l'organisme hôte est préférentiellement un homme ou un mammifère non humain, bien qu'il puisse aussi s'agir d'une plante.

En général, les complexes formés entre un acide nucléique d'intérêt et une composition lipophile ou une vésicule lipidique de l'invention sont présents dans une solution liquide appropriée, telle que de l'eau distillée stérile et apyrogène, en des quantités de complexes appropriées. La solution peut être utilisées telle quelle, ou encore contenir également un ou plusieurs agents stabilisants, tels que le Tween® (20, 40, 60 ou 80), du NaCl, ou encore du DMPE-PEG 5000.

La présente invention est encore relative à une composition pharmaceutique comprenant un complexe formé entre un acide nucléique d'intérêt et une composition lipophile ou une vésicule lipidique de l'invention, le cas échéant en association avec un ou plusieurs véhicules ou excipients physiologiquement compatibles.

De manière générale, les composés de formule (I) et les composés de formule (XI) définis dans la présente description font partie de l'invention.

En particulier, les composés lipophiles de formule (XI) sont nouveaux et font donc également partie de l'invention.

L'invention a donc aussi pour objet un composé lipophile de formule (XI) suivante : 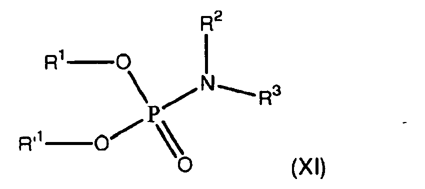 dans laquelle :
(i) R¹ et R'¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R² est une atome d'hydrogène ou une chaîne alkyle ayant de 1 à 4 atomes de carbone ;
(iii) R³ est un groupe de formule 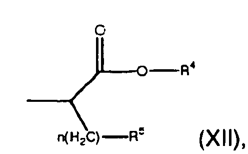 dans laquelle
   - R⁴ est un groupe alkyle ayant de 1 à 4 atomes de carbone ;
   - n est un entier égal à 1, 2, 3 ou 4 ; et
   - R⁵ est un groupe choisi parmi : 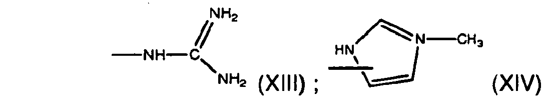
   et -NH₃.

De manière générale, les groupes R¹, R'¹, R², R³, R⁴ et R⁵ ont les significations définies précédemment dans la présente description.

Selon un premier mode de réalisation préféré, le groupe R⁵ possède la formule suivante :

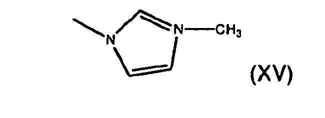

Selon un second mode de réalisation préféré, que les groupes R¹ et R'¹ sont identiques et représentent chacun la chaîne monoalcényle C_{18 :1}, dans lequel le premier nombre représente le nombre d'atomes de carbone dans la chaîne alcényle et le second nombre représente le nombre de double liaisons dans la chaîne alcényle.

L'invention a aussi pour objet un composé lipophile de formule (I) suivante : 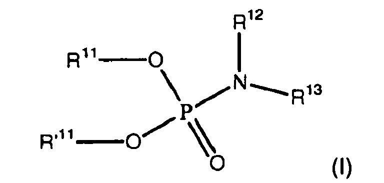
(i) R¹¹ et R,'¹¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R¹² est un atome d'hydrogène ou une chaîne alkyle ayant de 1 à 4 atomes de carbone ;
(iii) R¹³ est un groupe de formule 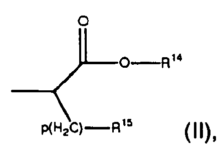 dans laquelle
   - R¹⁴ est un groupe alkyle ayant de 1 à 4 atomes de carbone ;
   - p est un entier égal à 1, 2, 3 ou 4 ; et
   - R¹⁵ est le groupe suivant :

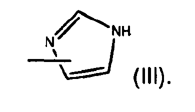

De manière générale, les groupes R¹¹, R'¹¹, R¹², R¹³, R¹⁴ et R¹⁵ ont les significations définies précédemment dans la présente description.

Selon un premier mode de réalisation préféré, le groupe R¹⁵ est choisi parmi les groupes suivants :

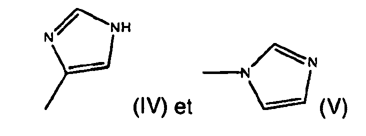

Selon un second mode de réalisation préféré, le groupe R¹⁶ est choisi parmi les groupes suivants :

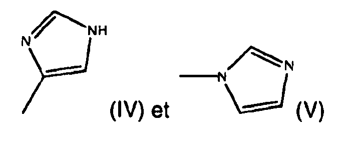

Selon un troisième mode de réalisation préféré, les groupes R¹¹ et R'¹¹ sont choisis parmi :
- le groupe tetradecyl,
- le groupe oleyl, et
- les groupes polyalcényl C_{18 :2} et C_{18 :3}, dans lequel le premier nombre représente le nombre d'atomes de carbone dans la chaîne alcényle et le second nombre représente le nombre de double liaisons dans la chaîne alcényle ;
- le groupe monoalcényle C_{18 :1}, dans lequel le premier nombre représente le nombre d'atomes de carbone dans la chaîne alcényle et le second nombre représente le nombre de double liaisons dans la chaîne alcényle.

Selon un quatrième mode de réalisation préféré, les groupes R¹¹ et R'¹¹ sont identiques.

Dans les composés de formule (I) et dans les composés de formule (XI), les parties - NR¹² R1³ et -NR²R³, respectivement, consistent en des dérivés d'acides aminés, en particulier en des dérivés d'acides aminés naturels. Cette caractéristique structurelle des composés de formule (I) et (XI) selon l'invention pourrait expliquer, au moins en partie, leurs propriétés cytotoxiques réduites, lorsque ces dérivés d'acides aminés sont combinés avec la présence d'un groupe de liaison («linker ») de type phosphoramidate.

Une autre caractéristique technique des composés de formule (XI) pour lesquels le groupe R³ est un groupe de formule (XII), est que le groupe ester -OR⁴, qui ne joue pas un rôle direct dans la formation de complexes entre ces composés lipophiles cationiques et le ou les acides nucléiques d'intérêt, est susceptible de posséder des fonctions avantageuses.

En premier lieu, le groupe ester -C(O)-OR⁴ possède une fonction d'accepteur de liaisons hydrogène, ce qui permet d'accroître les forces d'interaction entre le ou les acides nucléiques d'intérêt et le composé lipophile cationique de formule (XI), et donc de renforcer la cohésion des complexes composés lipophiles/acides nucléiques.

En second lieu, le groupe ester -C(O)-OR⁴ peut être hydrolyse après sont internalisation dans la cellule, en particulier via une réaction d'hydrolyse acide, du fait de l'environnement acide des endosomes, ou bien encore via une réaction d'hydrolyse enzymatique, du fait de la présence d'estérases dans les endosomes, étant entendu que l'hydrolyse peut être à la fois une hydrolyse acide pour certains des composés lipophiles cationiques et une hydrolyse enzymatique pour les autres composés lipophiles cationiques. Après hydrolyse du groupe ester, le composé lipophile, initialement cationique, devient un zwitterion, ce qui est susceptible d'améliorer le routage intracellulaire du composé lipophile et ainsi accroître sa capacité à transfecter efficacement es cellules hôtes humaines ou animales.

Des remarques similaires peuvent être faites pour les composés lipophiles neutres de formule (I) dans lesquels le groupe R¹³ est un groupe de formule (II), puisque le groupe ester - C(O)-OR¹⁴ peut être hydrolyse de la même manière que le groupe ester -C(O)-OR⁴ des composés de formule (XI). En milieu acide, ou en présence d'estérases, les composés de formule (I) deviennent des composés lipophiles anioniques, ce qui est également susceptible de favoriser la capacité de transfection d'une composition lipophile selon l'invention.

Des caractéristiques additionnelles des composés lipophiles de formules (I) et (XI), ainsi que de leur procédé de préparation, sont détaillées ci-dessous.

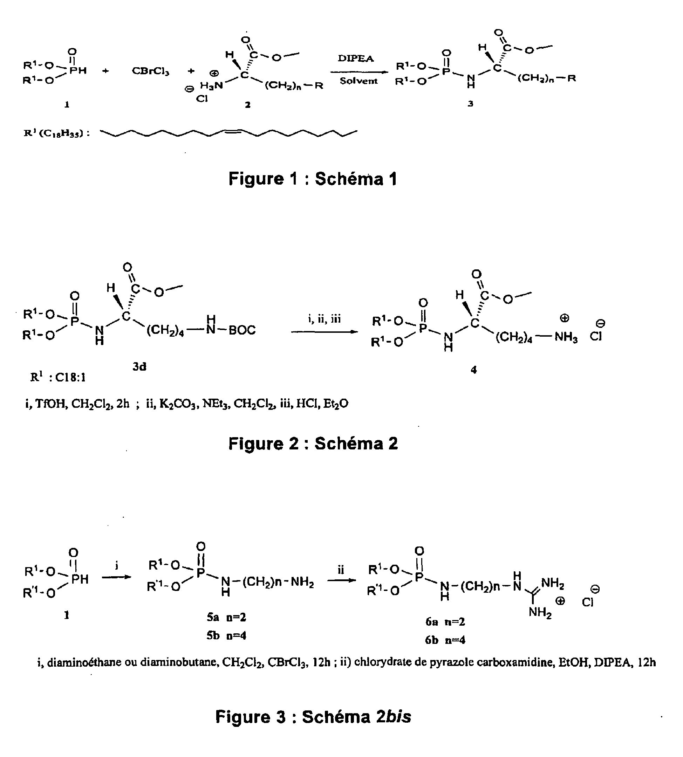

Dans le schéma 1 (Figure 1), DIPEA est la diisopropylethylamine, et R (ou R⁵) est le groupement basique latéral de l'aminoester qui, protoné au pH physiologique, permet la complexation de l'ADN. Parmi les aminoesters dérivés d'aminoacides naturels, ont été retenus les esters méthyliques de l'arginine, et de la lysine (pKa = 12,48 and 10,57 respectivement). L'homoarginine (pKa = 12,5) a également été retenue, pour des raisons de similarité avec l'arginine. A cause des propriétés particulières de l'histidine dues à la présence latérale du noyau imidazole, des composés comportant cet aminoester ont également été synthétisés. Ce noyau imidazole possède un pKa de 6,04, ce qui signifie qu'environ 5% du noyau imidazole est protoné au pH physiologique, et que les 95% restants pourront agir comme co-lipide neutre.

On a récapitulé dans le tableau 1 de la partie exemples, quelques modes de réalisation de vecteurs ainsi obtenus en appliquant le schéma 1 de synthèse représenté à la Figure 1. Les rendements sont donnés à titre indicatif et n'ont pas été optimisés.

Pour appliquer le même principe de synthèse à l'ester méthylique de la lysine, il a fallu utiliser l'aminoester protégé, la N-BOC lysine méthylester. Après phosphorylation, comme décrit dans le schéma 1 (Figure 1), on procède à une déprotection par l'acide trifluoroacétique, et l'on remplace ensuite l'anion triflate par un chlorure, pour obtenir *in fine* le phosphoramidate **4**, ainsi que décrit dans la figure 2.

D'autres phosphoramidates de formule (I) et (XI) ont été synthétisés, dont le groupe R¹³ représente un groupe de formule -(CH₂)_{q}-R¹⁶ ou dont le groupe R³ représente un groupe de formule -(CH₂)_{q}-R⁶. La figure 3 montre par exemple le principe de synthèse de composés **6** possédant un groupement guanidine, comme l'arginine. Il consiste à préparer les dérivés aminés **5** par condensation de diamines sur un phosphite lipidique, puis à guanidyler l'amine par un réactif de guanidylation comme la pyrazole-1-carboxamidine. La synthèse de ces derniers composés est illustrée dans la Figure 3. Dans la Figure 3, les références ont les significations suivantes : i) CBrCl₃, 2-diaminoethane or 1,4-diaminobutane, CH₂Cl₂, 20°C ii) 1H-pyrazole-1-carboxamidine.monochlorhydrate, DIPEA, ethanol, 79°C.

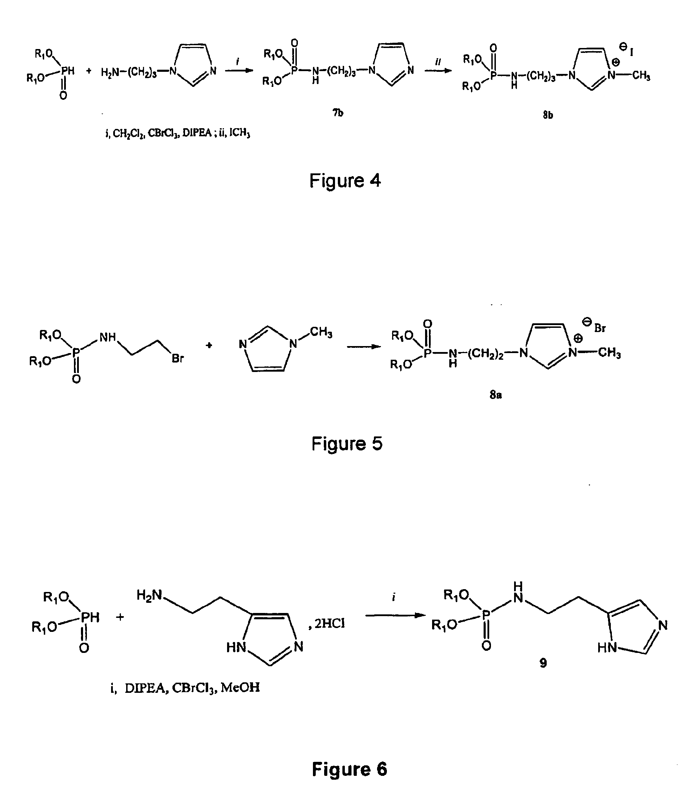

On a également préparé des phosphoramidates de formules (I) et (XI), dont le groupe R¹³ représente un groupe de formule -(CH₂)_{q}-R¹⁶ ou dont le groupe R³ représente un groupe de formule -(CH₂)_{q}-R⁶, qui possèdent un noyau imidazole, comme l'histidine, comme il est décrit dans la Figure 4. La Figure 4 illustre un exemple de préparation d'un phosphoramidate à noyau imidazole. Dans la figure 4, les références ont les significations suivantes : I i) CBrCl₃, DIPEA, 3-aminopropylimidazole, CH₂Cl₂, 20°C ii) CH₃l, excès, 20°C, 16h.

Comme le pKa de l'imidazole est d'environ 6, cet hétérocycle n'est que partiellement protoné au pH physiologique. Pour garantir une charge cationique permanente, on a également synthétisé les sels d'imidazolium correspondants **8**. Pour ce faire, on a condensé le N-méthyl imidazole sur un phosphoramidate bromé comme il est décrit dans la figure 5 (synthèse de **8a**), ou bien on a quaternarisé le phosphoramidate **7b** par l'iodure de méthyle (synthèse de **8b**), comme il est décrit dans la figure 4.

Dans ces exemples, le noyau imidazole est lié au reste de la molécule par l'un des atomes d'azote. On a synthétisé également un lipide **9** dont le noyau imidazole est lié au reste de la molécule par l'un des atomes de carbone du cycle. Pour ce faire, on a fait réagir l'histamine sur un phosphite lipidique, comme il est décrit dans la figure 6. Dans la figure 6, les références ont les significations suivantes : I i) CBrCl₃, DIPEA, MeOH, 20°C

On a ainsi synthétisé et évalué pour la première fois une nouvelle famille de phosphoramidates qui ont tous en commun leur partie lipidique (chaînes oléoïques, c'est-à-dire à 18 carbones et une insaturation centrale) et une partie polaire dérivée d'un amino- acide naturel ou d'une partie de ses constituants

Cette famille comprend donc des lipides cationiques et pour certains d'entre eux leurs précurseurs, comme le composé **7b**, qui est un lipide neutre.

La présente invention est aussi relative aux procédés de préparation des composés lipophiles de formules (I) et (XI) définis dans la présente description.

De manière générale, les composés de formule (I) pour lesquels le groupe R¹³ consiste en un groupe de formule (II) ; ainsi que les composés de formule (XI) pour lesquels le groupe R³ consiste en un groupe de formule (XII), peuvent être préparés selon le schéma 1 de procédé représenté sur la Figure 1.

Dans le schéma 1 représenté sur la Figure1 :
- pour la préparation des composés de formule (I) :
- les groupes R¹¹ et R'¹¹ correspondent aux deux groupes R' et R'¹, respectivement ;
- le groupe R¹⁴ correspond au groupe R⁴ ;
- le groupe R¹⁵ correspond au groupe R⁵ ;
- le groupe -(CH₂)ₚ- correspond au groupe -(CH₂)ₙ-
- pour la préparation des composés de formule (XI) :
- les groupes R¹ et R'¹ correspondent aux deux groupes R¹ et R'¹, respectivement ;
- le groupe R⁴ correspond au groupe R⁴ ;
- le groupe R⁵ correspond au groupe R⁵ ;
- le groupe -(CH₂)ₙ- correspond au groupe -(CH₂)ₙ.

Les composés de formule (XI) pour lesquels le groupe R³ consiste en un groupe de formule -(CH₂)ₒ-R⁶, et plus spécifiquement ceux des composés pour lesquels le groupe R⁵ consiste en un groupe de formule (XIII), peuvent être préparés selon le Schéma 2 de procédé représenté sur la Figure 2.

Les composés de formule (XI) pour lesquels le groupe R³ consiste en un groupe de formule -(CH₂)ₒ-R⁶, et plus spécifiquement ceux des composés pour lesquels le groupe R⁵ consiste en un groupe de formule (XIV), peuvent être préparés, à partir d'un composé de formule (I), selon le Schéma 4 de procédé représenté sur la Figure 3, ou encore selon le Schéma 5 représenté sur la Figure 4.

Les composés de formule (I) lesquels le groupe R¹³ consiste en un groupe de formule - (CH₂)ₒ-R¹⁶, et plus spécifiquement ceux des composés pour lesquels le groupe R¹⁵ consiste en un groupe de formule (IV), peuvent être préparés selon le Schéma 3 de procédé représenté sur la Figure 5.

Les composés de formule (I) lesquels le groupe R¹³ consiste en un groupe de formule - (CH₂)ₒ-R¹⁶, et plus spécifiquement ceux des composés pour lesquels le groupe R¹⁵ consiste en un groupe de formule (V), peuvent être préparés selon le Schéma 6 de procédé représenté sur la Figure 6.

Les protocoles détaillés de synthèse des composés de formule (I) et des composés de formules (XI) sont décrits dans les exemples.

De manière générale, les composés lipophiles cationiques de formule (XI) peuvent se présenter sous la forme de sels avec un anion, c'est à dire sous la forme d'un sel avec toutes molécules organiques ou minérales qui est chargée négativement dans une solution au pH physiologique. Selon un premier aspect, les composés lipophiles de formule (XI) peuvent être préparés sous la forme de sels avec un anion, ledit anion pouvant être choisi parmi CF₃CO₂⁻, CF₃SO₃⁻, HSO₄⁻ et un halogène. Ledit halogène peut être choisi parmi Cl⁻, Br⁻ et I⁻. Selon un second aspect, lorsqu'un composé de formule (XI) est ajouté à une solution saline à un pH neutre physiologique, il forme un sel avec les anions présents dans ladite solution saline. Par exemple, dans un milieu de culture cellulaire, ou bien encore dans un fluide corporel d'un organisme humain ou animal, un composé lipophile cationique de formule (XI) est retrouvé classiquement sous la forme d'un sel de chlorure.

La présente invention est en outre illustrée, sans pour autant être limitée, par les exemples suivants.

### EXEMPLES

### Protocoles de synthèse des composés de formule (I) et (XI)

### Exemple 1 : Synthèse d'un phosphoramidate de dioleoyle dérivé de l'ester méthylique de l'histidine

On mélange 2,91 g de dioléylphosphite (5 mmol), 920,35 mg d'histidine méthyl ester dichlorhydrate (5 mmol), 15 mL de MeOH et 550 µL de CBrCl₃ (5,5 mmol). Le mélange est placé à une température inférieure à 5°C dans un bain glace/acétone. On ajoute ensuite 2.6 mL de DIPEA (15 mmol) et on agite à cette température une heure puis une nuit à température ambiante.

Une purification par chromatographie sur colonne de gel de silice avec élution par un mélange CHCl₃/MeOH (90/10) permet d'isoler une huile jaune clair avec un rendement de 29% (m=1,08g).

La structure du composé est vérifiée par RMN du proton, du phosphore 31, du carbone 13 et spectrométrie de masse :
**RMN ¹H en ppm (CDCl₃)**
0,86 (t, 6H, CH₃, ³J_{H-H}= 6,6Hz) ; 1,26 (m, 44H, CH₂) ; 1,65 (m, 4H, CH₂ □-O) ; 1,99 (m, 8H, CH₂ o-CH=CH) ; 3,08 (d, 2H, CH₂Im, ³J_{H-H} = 5,3 Hz) ; 3,71 (s, 3H, OCH₃) ; 3, 80 (t, NH, 10Hz) ; 3,98 (m, 4H, CH₂ □-O, ³J_{H-H} = ³J_{P-H}= 6,4Hz) ; 4,12 (m, CH(NH)) ; 5,32 (m, 4H, CH=CH) ; 6,80 (s, H¹) ; 7,53 (s, H²)
**RMN ¹³C en ppm (CDCl₃) :**
14,1 (s, CH₃) ; entre 22,6 et 32,0 (plusieurs singulets, CH₂) ; 30,3 (d, CH₂ □-O, ³J_{P·C} = 6,2 Hz) ; 30,9 (s, CH₂Im) ; 52,3 (s, OCH₃) ; 54,3 (s, CH(NH)) ; 66,1 (d, CH₂ □-O,²J_{P-C} = 6,6 Hz) ; 115,4 (s, C¹) ; 129,6 (s, CH=CH) ; 129,7 (s, CH=CH) ; 131,5 (s, C_{quaternaire}) ; 134,8 (s, C²) ; 172,8 (s, CO)
**RMN ³¹P en ppm (CDCl₃) :**
7,6 (s)
**Spectrométrie de masse** :
ESI pour C₄₃H₈₁N₃O₅P, [M + H]⁺ calculé 750,59139, trouvé 750,5905

### Exemple 2 : Synthèse d'un phosphoramidate de dioleoyle derivé de l'ester méthylique de l'arginine

On mélange 2,80 g de dioléylphosphite (4,8 mmol), 1,27 g d'arginine méthylester dichlorhydrate (4,8 mmol), 15 mL de MeOH et 550 µL de CBrCl₃ (5,5 mmol). Le mélange est placé à une température inférieure à 5°C dans un bain glace/acétone. On ajoute ensuite 2,50 mL de DIPEA (14,4 mmol) et on agite à cette température une heure puis une nuit à température ambiante.

Une purification par chromatographie sur colonne de gel de silice avec élution par un mélange CHCl₃/MeOH (90/10) permet d'isoler une huile jaune clair avec un rendement de 31%

La structure du composé est vérifiée par RMN du proton, du phosphore 31, du carbone 13 et spectrométrie de masse:
**RMN ¹H en ppm (DMSO) :**
0,83 (t, 6H, CH₃, ³J_{H·H} = 6,6Hz) ; 1,23 (m, 44H, CH₂) ; 1,53 (m, 4H, CH₂ □-O); 1,96 (m, 8H, CH₂ α-CH=CH) ; 3,95 (m, 4H, CH₂ α-O, ³J_{H-H} = ³J_{P-H}= 6,4Hz) ; 5,31 (m, 4H, CH=CH), 1,52 (m, 2H²) ; 1,65 (m, 2H¹) ; 3,10 (m, 2H³) ; 3,60 (m, CH) ; 3,61 (s, OCH₃) ; 5,42 (t, NH¹) ; 7,80 (m, NH²)
**RMN ¹³C en ppm (DMSO) :**
13,8 (s, CH₃) ; entre 22,0 et 31,3 (plusieurs singulets, CH₂) ; 25,1 (s, C²) ; 29,0 (d, CH₂ □-O, ³J_{P-C} = 6,2 Hz) ; 30,0 (s, C¹) ; 39,9 (s, C³) ; 51,6 (s, OCH₃) ; 53,6 (s, CH), 65,3 (d, CH₂ [□-O,²J_{P-C} = 6,6 Hz) ; 129,5 (s, CH=CH) ; 129,9 (s, CH=CH) ; 156,8 (s, C⁴) ; 173,5 (s, CO₂)
**RMN ³¹P en ppm (CDCl₃) :**
8,8 (s)
**Spectrométrie de masse :**
ESI pour C₄₃ H₈₆N₄O₅P, [M + H]⁺, calculé 769,63359, trouvé 769,6338

### Exemple 3 : Synthèse d'un phosphoramidate de dioleoyle derivé de l'ester méthylique de l'homoarginine

On mélange 2,80 g de dioléylphosphite (4,8 mmol), 1,34 g d'homoarginine méthylestes dichlorhydrate (4,8 mmol), 15 mL de MeOH et 550 µL de CBrCl₃ (5,5 mmol). Le mélange est placé à une température inférieure à 5°C dans un bain glace/acétone. On ajoute ensuite 2,50 mL de DIPEA (14,4 mmol) et on agite à cette température une heure puis une nuit à température ambiante.

Une purification par chromatographie sur colonne de gel de silice avec élution par un mélange CHCl₃/MeOH (90/10) permet d'isoler une huile jaune clair avec un rendement de 30%

La structure du composé est vérifiée par RMN du proton, du phosphore 31, du carbone 13 et spectrométrie de masse :
**RMN ¹H en ppm (DMSO) :**
0,83 (t, 6H, CH₃, ³J_{H-H} = 6,6Hz) ; 1,23 (m, 44H, CH₂) ; 1,45 (m, 2H³) ; 1,50 (m, 2H²) ; 1,53 (m, 4H, CH₂ □-O) ; 1,65 (m, 2H¹) ; 1,96 (m, 8H, CH₂ α-CH=CH) ; 3,06 (m, 2H⁴) ; 3,54 (m, CH) ; 3,62 (s, OCH₃) ; 3,95 (m, 4H, CH₂ α O, ³J_{H-H} = ³J_{P-H} = 6,4Hz) ; 5,31 (m, 4H, CH=CH) ; 5,38 (m, NH¹) ; 7,65 (m, NH²)
**RMN ¹³C** en **ppm (DMSO) :**
13,8 (s, CH₃) ; entre 22,0 et 31,3 (plusieurs singulets, CH₂) ; 21,3 (s, C²) ; 27,8 (s, C³) ; 29,0 (d, CH₂ □-O, ³J_{P-C} = 6,2 Hz) ; 29,4 (s, C¹) ; 40,7 (s, C⁴) ; 51,4 (s, OCH₃) ; 54,5 (s, CH) ; 65,3 (d, CH₂ □-O, ²J_{P-C} = 6,6 Hz) ; 129,5 (s, CH=CH) ; 129,9 (s, CH=CH) ; 156,8 (s, C⁵) ; 173,5 (s, CO₂)
**RMN ³¹P en ppm (DMSO) :**
8,9 (s)
**Spectrométrie de masse** :
ESI pour C₄₄H₈₈N₄O₅P, [M + H]⁺, calculé 783,64924, trouvé 783,6484

### Exemple 4 : Synthèse d'un phosphoramidate de dioleoyle derivé de l'ester méthylique de la lysine

On mélange 844 mg de dioléylphosphite (1,45 mmol), 430 mg de Boc-Lysine méthylester chlorhydrate (1,45 mmol), 15 mL de CHCl₃ et 200 µL de CBrCl₃ (2 mmol). Le mélange est placé à une température inférieure à 5°C dans un bain glace/acétone. On ajoute ensuite 510 µL de DIPEA (2,90 mmol) et on agite à cette température une heure puis une nuit à température ambiante.

Après évaporation des solvants, le mélange est repris avec de l'éther et les sels de DIPEA précipités sont éliminés par filtration. (Rdt 90%)
On ajoute ensuite 5 mL de CH₂Cl₂ et 5 mL d'acide trifluoroacétique et l'agitation est maintenue durant deux heures.

Après évaporation des solvants, le composé est solubilisé dans 10 mL de CH₂Cl₂, un excès de carbonate de potassium et une goutte de Et₃N sont ajoutés. L'agitation est maintenue durant trois heures. Après filtration, évaporation, addition de 10 mL de CH₂Cl₂ et d'un excès de HCl (en solution 2N dans l'éther), on maintient trente minutes sous agitation.

Une purification par chromatographie sur colonne de gel de silice avec élution par un mélange CHCl₃/MeOH (90/10) permet d'isoler une huile jaune clair avec un rendement de 70%.

La structure du composé est vérifiée par RMN du proton, du phosphore 31, du carbone 13 et spectrométrie de masse :
**RMN ¹H en ppm (DMSO) :**
: 0,83 (t, 6H, CH₃, ³J_{H-H} = 6,6Hz) ; 1,23 (m, 44H, CH₂) ; 1,35 (m, 2H²) ; 1,53 (m, 4H, CH₂ □-O) ;
1,55 (m, 2H³); 1,60 (m, 2H¹) ; 1,96 (m, 8H, CH₂ □-CH=CH) 2,70 (t, 2H⁴, ³J_{H-H} = 7,4 Hz) ; 3,55 (m, CH) ; 3,71 (s, OCH₃) ; 3,95 (m, 4H, CH₂ α-O, ³J_{H-H} = ³J_{P-H} = 6,4Hz) ; 5,31 (m, 4H, CH=CH) ; 5,35 (m, NH)
**RMN ¹³C en ppm (DMSO) :**
13,8 (s, CH₃) ; entre 22,0 et 31,3 (plusieurs singulets, CH₂) ; 25,0 (s, C²) ; 26,5 (s, C³) ; 28,8 (s, C') ; 29,0 (d, CH₂ □-O, ³J_{P-C} = 6,2 Hz) ; 38,5 (s, C⁴) ; 51,6 (s, OCH₃) ; 53,9 (s, CH) ; 65,3 (d, CH₂ α-O, ²J_{P-C} = 6,6 Hz) ; 129,5 (s, CH=CH) ; 129,9 (s, CH=CH chaînes grasses) ; 173,6 (s, CO₂)
**RMN ³¹P en ppm (DMSO) :**
8,7 (s)
**Spectrométrie de masse :**
ESI pour C₄₃H₈₆N₂O₅P, [M + H]⁺, calculé 741,62744 trouvé 741,6262

### Exemple 5 : Synthèse de l'iodure de 3-propylméthylimidazolium phosphoramidate de dioleoyle :

On mélange 2,91 g de dioléylphosphite (5 mmol), 590 µL d'aminopropylimidazole (5 mmol), 15 mL de CH₂Cl₂ et 550 µL de CBrCl₃ (5,5 mmol) en maintenant la température inférieure à 5°C dans un bain glace/acétone. On ajoute ensuite 960 µL de DIPEA (5,5 mmol) et on agite à cette température une heure puis une heure à température ambiante.

Après évaporation des solvants, le mélange est repris avec de l'éther et les sels de DIPEA précipités sont éliminés par filtration.
Après purification sur gel de silice (éluant CHCl₃/MeOH (90/10)), le phosphoramidate est obtenu avec 82% de rendement sous forme d'une huile jaune pâle.

La structure du composé intermédiaire est vérifiée par RMN du proton, du phosphore 31 et du carbone 13, puis on procède à la quaternarisation de la façon suivante :

On solubilise 2,8 g du composé 5 (4 mmol) dans un large excès de ICH₃ (3 mL) et on agite à température ambiante pendant 16 heures. Après évaporation une huile orange est récupérée. Le rendement est quantitatif.

La structure du composé est vérifiée par RMN du proton, du phosphore 31, du carbone 13 et spectrométrie de masse :
**RMN ¹H en ppm (CD₃OD) :**
0.89 (t, 6H, CH₃ , ³J_{H-H} = 6.6Hz) ; 1.28 (m, 44H, CH₂) ; 1.65 (m, 4H, CH2 □-O) ; 2.00 (m, 8H, CH₂ □-CH=CH) ; 2.03 (m, 2H²) ; 2.94 (m, 2H') ; 3.67 (s, 3H, CH₃) ; 3.97 (m, 4H, CH₂ □-O, ³J_{H-H} = ³J_{P-H} = 6.4Hz) ; 4.30 (t, 2H³,) ; 5.33 (m, 4H, CH=CH) ; 7.57 (s, CH²) ; 7.64 (s, CH³)
**RMN ¹³C en ppm (CD₃OD) :**
14.5 (s, 2 CH₃) ; entre 23.7 et 33.6 (plusieurs singulets, CH₂) ; 26.7 (d, 2 CH₂ □ O, ³J_{P-C} = 6.2 Hz) ; 28.3 (s, C²) ; 36.5 (s, CH₃) ; 38.5 (s, C¹) ; 48.0 (s, C³) ; 67.9 (d, 2 CH₂ α-O, ²J_{P-C} = 6.6 Hz) ; 123.8 (s, C²) ; 125.1 (s, C³) ; 130.7 (s, CH=CH) ; 130.9 (s, CH=CH) ; 131,8 (s, C¹)
**RMN ³¹P en ppm (CD₃OD) :**
9.9 (s)
**Spectrométrie de masse :**
ESI pour C₄₃H₈₃N₃O₃P, [M + H]⁺**,** calculé 720,61721, trouvé 720,6143

### Exemple 6 : Synthèse d'un phosphoramidate de dioleoyle derivé de l'histamine

On mélange 2,91 g de dioléylphosphite (5 mmol), 920,3 mg d'histamine dichlorhydrate (5 mmol), 15 mL de MeOH et 550 µL de CBrCL₃ (5,5 mmol). Le mélange est placé à une température inférieure à 5°C dans un bain glace/acétone. On ajoute ensuite 2,6 mL de DIPEA (15 mmol) et on agite à cette température une heure puis une nuit à température ambiante.

Une purification par chromatographie sur colonne de gel de silice avec élution par un mélange CHCl₃/MeOH (90/10) permet d'isoler une huile jaune clair avec un rendement de 30%.

La structure du composé est vérifiée par RMN du proton, du phosphore 31, du carbone 13 et spectrométrie de masse :
**RMN ¹H en ppm (CDCl₃) :**
0,86 (t, 6H, CH₃, ³J_{H-H} = 6,6Hz) ; 1,26 (m, 44H, CH₂) ; 1,65 (m, 4H, CH₂ □-O) ; 1,99 (m, 8H, CH₂ □-CH=CH) ; 2,79 (t, 2H, CH₂Im, ³J_{H-H} = 5,5 Hz) ; 3,13 (m, NH) 3,19 (m, 2H, CH₂(NH)) ; 3,98 (m, 4H, CH₂ α-O, ³J_{H-H} = ³J_{P-H} = 6,4Hz) ; 5,32 (m, 4H, CH=CH) ; 6,82 (s, H¹) ; 7,54 (s, H²)
**RMN ¹³C en ppm (CDCl₃) :**
14,1 (s, CH₃) ; entre 22,6 et 32,0 (plusieurs singulets, CH₂) ; 30,3 (d, CH₂ □-O_{.} ³J_{P-C} = 6,2 Hz) ; 29,1 (s, CH₂Im) ; 41,4 (s, CH₂(NH)) ; 66,1 (d, CH₂ α-O, ²J_{P-C} = 6,6 Hz) ; 116,0 (s, C¹) ; 129,6 (s, CH=CH) 129,7 (s, CH=CH) ; 131,1 (s, C_{quaternaire}) ; 135,1 (s, C²)
**RMN ³¹P en ppm (CDCl₃) :**
9,7 (s)
**Spectrométrie de masse :**
**28 :** ESI pour C₄₁H₇₉N₃O₃P, [M + H]⁺ calculé 692,58591 trouvé 692,5854

### Exemple 7 : Synthèse du 2-éthylguanidinium phosphoramidate de dioleoyle

On réalise dans un premier temps la synthèse d'un aminophosphoramidate intermédiaire de la façon suivante :

On mélange 1,5 g de dioléylphosphite (2,6 mmol), 1,7 mL de diaminoéthane (26 mmol), 10 mL de CH₂Cl₂, puis on ajoute 260 µL de CBrCL₃ (2,6 mmol) goutte à goutte. L'agitation est maintenue toute la nuit.

Après lavage à l'eau, séchage sur sulfate de magnésium et évaporation du solvant une huile de couleur jaune est récupérée avec un rendement de 85%.

La structure du composé est vérifiée par RMN du proton, du phosphore 31, du carbone 13 et spectrométrie de masse :
**RMN ¹H en ppm (CDCl₃) :**
0,86 (t, 6H, CH₃, ³J_{H-H} = 6,6Hz) ; 1,26 (m, 44H, CH₂), 1,65 (m, 4H, CH₂ □-O) ; 1,99 (m, 8H, CH₂ α-CH=CH) ; 2,64 (m, NH₂) ; 2,78 (t, 2H², ³J_{H-H} = 5,5 Hz) ; 2,94 (m, 2H') ; 3,33 (m, NH); 3,98 (m, 4H, CH₂ α-O, ³J_{H-H} = ³J_{P-H} = 6,4Hz) ; 5,32 (m, 4H, CH=CH)
**RMN ¹³C en ppm (CDCl₃) :**
14,1 (s, CH₃) ; entre 22,6 et 32,0 (plusieurs singulets, CH₂) ; 30,3 (d, CH₂ □-O, ³J_{P-C} = 6,2 Hz) ; 42,7 (s, C²) ; 43,7 (s, C¹) ; 66,1 (d, CH₂ α-O, ²J_{P-C} = 6,6 Hz) ; 129,6 (s, CH=CH) ; 129,7 (s, CH=CH)
**RMN ³¹P en ppm (CDCl₃) :**
10,0 (s)
**Spectrométrie de masse :**
ESI pour C₃₈H₇₈N₂O₃P, [M + H]⁺, calculé 641,57501 trouvé 641,5739

On procède ensuite à la guanidylation de l'amine terminale de la façon suivante :

On mélange 1,34 g du composé intermédiaire précédent, 309 mg de chlorhydrate de pyrazole carboxamidine (2,1 mmol), 10 mL d'éthanol absolu et 367 µL de DIPEA (2,1 mmol) et on chauffe à reflux pendant une nuit.

Après évaporation du solvant, un lavage basique, une extraction au CH₂Cl₂ puis un séchage sur MgSO₄ sont effectués.

Une purification par chromatographie sur colonne de gel de silice avec élution par un mélange CHCl₃/MeOH (90/10) permet d'isoler une huile jaune clair avec un rendement de 70%

La structure du composé est vérifiée par RMN du proton, du phosphore 31, du carbone 13 et spectrométrie de masse :
**RMN ¹H en ppm (CD₃OD) :**
0,84 (t, 6H, CH₃, ³J_{H-H} = 6,6Hz) ; 1,24 (m, 44H, CH₂) ; 1,55 (m, 4H, CH₂ □-O) ; 1,96 (m, 8H, CH₂ α-CH=CH) ; 2,85 (m, 2H¹) ; 3,14 (m, 2H²) ; 3,87 (m, 4H, CH₂ α-O, ³J_{H-H} = ³J_{P-H} = 6,4Hz) ; 4,96 (m, NH) ; 5,32 (m, 4H, CH=CH) ; 7,54 (m, NH)
**RMN ¹³C en ppm (CD₃OD) :**
14,1 (s, CH₃) ; entre 22,6 et 32,0 (plusieurs singulets, CH₂) 26,6 (d, CH₂ □-O, ³J_{P-C} = 6,2 Hz) ; 39,9 (s, C¹) ; 42,0 (s, C²) ; 65,4 (d, CH₂ α-O, ²J_{P-C} = 6,6 Hz) ; 129,6 (s, CH=CH) ; 129,7 (s, CH=CH) ; 157,8 (s, C₄)
**RMN ³¹P en ppm (CD₃OD) :**
9,9 (s)
**Spectrométrie de masse :**
ESI pour C₃₉H₈₀NO₃P, [M + H]⁺, calculé 683,59681, trouvé 683,5967

**Tableau 1: Synthèse des phosphoramidate-aminoesters 3a-d.**

| Entrée | Substrat | R | n | DIPEA | Solvant | N° produit | Rendement % |
|---|---|---|---|---|---|---|---|
| 1 | **2a** (arginine methylester) | 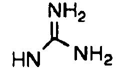 | 3 | 3eq | MeOH | 3a | 31 |
| 2 | **2b** (homoarginine methyl ester) | 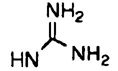 | 4 | 3 eq | MeOH | 3b | 30 |
| 3 | **2c** (histidine methylester) | 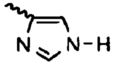 | 1 | 3 eq | MeOH | 3c | 29 |
| 4 | **2d** (N-BOC lysine methylester) | - NH - BOC | 4 | 2 eq | CHCl₃ | 3d | 90 |

### Exemple 2 : Evaluation de la capacité de transfection de compositions lipophiles de l'invention.

### A. Matériel et Méthodes

### A.1. Liposomes

Un film lipidique est préparé dans un ballon stérile sous azote en évaporant à sec 1 ml de lipide cationique à 10,8 mM ou d'un mélange lipide cationique/lipide neutre (rapport molaire 1:1) dans l'éthanol. Le film est ensuite hydraté avec 1 ml de tampon hepès 10 mM, pH 7,4, la solution agitée fortement pendant 3 min puis mise au repos à 4°C. Après 2h, la solution est soniquée pendant 15 min dans un bain ultrason à 37 kHz (Bioblock ultrasonic bath, Bioblock Scientific, IIIkirch, France).

### A.2. Complexes ADN/liposomes

18 µl d'une solution de liposomes à 5,4 mM sont dilués dans 200 µl de tampon hepès 10 mM, pH 7,4. Après 15 min, 7,5 µg d'un plasmide codant le gene de la luciferase (pTG11033 ; 9514 bp, Transgene S.A., Strasbourg, France) dans 20µl de tampon hepès 10 mM, pH 7,4, sont ajoutés aux liposomes et le mélange est laissé au repos pendant 30 min. La solution contenant les complexes ADN/liposomes est ajustée à 1,5 ml avec du milieu de culture sans sérum et à 0,15M NaCl avec une solution de NaCl à 5M.

### A.3. Transfections

Deux jours avant la transfection, les cellules 293T7 (fibroblastes embryonaires de reins humains) sont ensemencées à raison de 1 x 10⁵ cellules dans 1 ml de milieu de culture dans une plaque de 24 puits. Le jour de la transfection, les cellules sont à 80% de confluence. Les cellules sont lavées deux fois avec du milieu sans sérum avant d'ajouter 2,5 µg d'ADN sous forme de complexes ADN/liposomes. Après 4 h d'incubation à 37 °C, le milieu est éliminé et les cellules sont mises en culture pendant 48h avec du milieu de culture complet.

### A.4. Mesure de l'expression de la luciferase dans les cellules transfectées (Protocole adapté de De Wet (1987).

Le milieu de culture des cellules est enlevé et chaque puits est rincé avec 500µL de PBS. Les cellules sont détachées avec 500µL du PBS + trypsine par puits pendant 5 min à 37°C. La suspension cellulaire est ensuite centrifugée pendant 5 min à 250g (1500 rpm) à 20°C. On ajoute 400µL de tampons de lyse (CCLR, Promega) sur le culot cellulaire qu'on laisse incuber sur glace pendant 15 min. Après avoir agité au Vortex, on centrifuge à 12 000g pendant 2 min à 4°C. L'activité luciférase contenue dans 20 µL de surnageant est mesurée au luminomètre (Bertold Lumat LB 9501) pendant 10 secondes après injection de 100µL de luciférine (Luciférase Assay Reagent, Promega). L'émission de lumière est convertie en unité arbitraire que l'on rapporte à la quantité de protéines de l'échantillon dosée par la méthode de l'acide bicinchoninique.

### A.5. Dosage de protéine :

La quantité de protéines dans le lysat est évaluée par la méthode du BCA (H.Hill et G.Straka, 1998). Les protéines sont mises en présence d'acide bicinchoninique (BCA) et d'ions cuivre en milieu alcalin. Les ions cuivreux générés par leur réduction par les protéines forment avec le BCA des complexes stables colorés. La coloration est objectivée par dosage spectrophotométrique (562 nm). Après établissement d'une gamme étalon réalisée avec de l'albumine de sérum bovin on détermine la masse de protéine contenue dans les lysats cellulaires

### A.6. Transfection dans le tendon d'Achille lésé de rat.

L'animal anesthésié dans les conditions réglementaires est ensuite opéré sous une hôte. Après avoir nettoyée soigneusement la patte à l'éthanol 70%, on incise la peau longitudinalement à l'aide d'une lame de scalpel courbe taille 12 (Swann Morton, Sheffield, Angleterre) depuis le 1/3 supérieur du muscle gastrocnémien jusqu'au calcanéum (talon). La gaine entourant le tendon d'Achille est ensuite incisée. La lésion tendineuse est effectuée en faisant une entaille légère du tendon sur environ 3mm de façon longitudinale au tiers moyen inférieur.

On procède ensuite à l'injection de l'ADN (formulé avec les vecteurs ou nu) contenu dans un volume de 40µl à l'aide d'une seringue à insuline de 22G (Omnican, MWR). La plaie est ensuite suturée à l'aide de fil de suture (Prolene, FS-2,19mm, Ethicon, Centravet). La patte est opérée, puis recouverte de Vétedine© (antiseptique/antifongique) (Vétoquinol, Centravet). Les rats opérés sont ensuite placés dans une cage de réveil.

### A.7. Mesure de l'expression de la luciferase dans les tendons transfectés

Après euthanasie des rats au CO₂, on place rapidement ces derniers sur glace. Le tendon est rapidement prélevé après incision et plongé dans du HBSS froid. On essuie les tendons, que l'on plonge dans l'azote liquide. Chaque tendon est alors broyé dans un mortier à l'aide d'un pilon. Les paillettes ainsi produites sont transférées dans un tube Eppendorf contenant 500µL de tampon de lyse (CCLR, Promega). Après une incubation de 3h sur glace, on centrifuge 30 secondes à 13000 g. On transfère ensuite le surnageant dans un tube luminomètre et on procède à la lecture RLU comme précédemment. L'émission de lumière est rapportée à la quantité de protéines présente dans le lysat.

### A.8. Culture primaire de ténocytes

La culture primaire de ténocytes est réalisée à partir d'explants de tendons d'Achille de rats « Wistar Han adulte » d'environ 250g. Après euthanasie, les rats sont immédiatement placés sur la glace. Sous une hôte à flux laminaire, on procède à l'extraction des tendons d'Achille à l'aide d'outils chirurgicaux stériles après avoir soigneusement nettoyé les pattes arrières à l'alcool. Les tendons sont immédiatement plongés dans du HBSS (Hanks' Balanced Salt Solution) stérile enrichi en antibiotiques (pénicilline 250U/mL, streptomycine 250µg/mL, kanamycine 100µg/mL) et gardés sur la glace. Les tendons sont rincés 3 fois de suite avec du HBSS enrichi en antibiotiques et mis en présence de 1mL de HBSS dans une boite de pétri. On enlève, à l'aide d'une lame de scalpel stérile, les parties non désirées du tendon telles que des morceaux de muscle, des tissus gras ou nécrosés. On transfère les parties désirées dans une autre boite de pétri en présence de HBSS et on les dissèque en petits explants de 0,3 à 0,5 mm de coté. Les explants sont récupérés à l'aide d'une pipette et mis dans 1 mL de HBSS dans un tube Falcon de 15 mL. On centrifuge à 250 g pendant 5 minutes. Arès avoir jeté le surnageant, les explants sont récupérés dans du milieu complet (DMEM complété avec 10% de sérum de veau foetal décomplémenté, de la vitamine C (44µg/mL), d'un mélange Pénicilline/streptomycine (250U/mL), kanamycine (91µg/mL) (Sigma), gentamycine (91µg/mL)). Au bout de 15 jours, des ténocytes apparaissent autour des explants. Selon la densité des explants, les ténocytes atteignent la confluence dans les deux à trois semaines suivantes. Les cellules sont ensuite décollées à l'aide de PBS contenant de l'EDTA 2mM et de la trypsine 2,5 µg/ml. Les boîtes de culture sont ensemencées à raison de 100 000 cellules par cm².

### B. Résultats

### B.1. Transfection dans cellules humaines

Sur l'ensemble des tests de transfection des cellules de la lignée 293T, il a été observé que les lipides 3a, 3b et 4 sont plus efficaces quand ils sont associés avec les co-lipides **3c** ou **7b** qu'avec la DOPE. Dans le Tableau 2 ci-dessous, on a rassemblé quelques exemples de ces efficacités, en reportant les valeurs de luminescence observées (exprimées en « Total Relative Light Units (TRLU) » comme indiqué dans le protocole ci-dessus), et l'on a indiqué entre parenthèses le gain d'efficacité par rapport à la DOPE.

**Tableau 2**

| Co-lipide Lipide cationique | - | DOPE | 3c | 7b |
|---|---|---|---|---|
| 3a | 7.10⁸ | 5.10⁸ | 5.10⁸(100) | 10⁶(0.2) |
| 3b | 9.10⁸ | 3.10⁶ | 3.10⁹ (1000) | 3.10⁷(10) |
| 4 | 2.10⁷ | 3.10⁷ | 5.10⁷(1.6) | 4.10⁷(1.3) |

On observe donc qu'à l'exception de l'association **3a/7b**, il y a avantage à utiliser les co-lipides **3c** et **7b** plutôt que la DOPE.

L'homme de l'art sait que les résultats des évaluations biologiques in vitro ne sont pas toujours en concordance avec les évaluations in vivo. Les demandeurs ont donc procédé à une série d'évaluations in vivo dans le cas de tentatives de réparation de tendons défectueux chez le rat.

### B.2. Transfection dans des tendons d'Achile avec une lésion tendineuse expérimentale

Les résultats sont représentés sur la Figure 7.

L'efficacité de transfection après 24h est forte avec **8a/9** avec un niveau proche de celui obtenu avec l'ADN nu ou complexé avec le JetPEI. L'intérêt du vecteur **8a/9** réside dans le fait qu'il permet une expression importante de la luciférase (∼5 10⁶ RLU/mg) et que celle-ci ne chute que d'un facteur 5 au bout de 3^{éme} jour et que ce niveau est maintenu jusqu'au 6^{éme} jour alors qu'avec l'ADN nu elle chute encore. L'expression du gène obtenue avec le JetPEI décroît de façon spectaculaire dès le 3^{ème} jour (100 fois moins). Les résultats montrent qu'il y a avantage à utiliser le lipide cationique 8a associé au co-lipide 9 pour transfecter efficacement le tendon d'Achille.

### Exemple 3 : Cytotoxicité réduite d'une composition lipophile selon l'invention

### A. Matériel et Méthodes

### Test de cytotoxicité :

Quarante huit heures après la transfection des cellules in vitro, on teste leur viabilité. Pour cela, on ajoute 50 µL de MTT (3-(4,5diméthyl-2thiazolyl)-2,5diphényl-2H-tétrazolium bromide) à 5mg/mL par puits de culture suivi d'une incubation de 4h à 37°C. Le milieu de chaque puits est enlevé dans des tubes Eppendorf. Chaque puits est ensuite rincé au PBS (2 fois 500 µL ). Puis, on ajoute successivement 1mL d'isopropanol acidifié et 200µL de SDS (3%) dans chaque puits. Après 30 min d'incubation à température ambiante, les suspensions cellulaires sont récupérées dans des tubes Eppendorf de 1,5mL et solubilisées au Vortex. Enfin, on prélève 3 fois 200 µL de chaque tube qu'on distribue sur une plaque de 96 puits et la lecture de l'absorbance est effectuée à 570 nm.

### B. Résultats

On observe également une absence quasi-totale de cytotoxicité, comme il est indiqué sur la Figure 8, ou l'on compare le couple **8a/9** à un polymère du commerce (Jet PEI) également efficace mais présentant plus de cytotoxicité.

### Exemple 4 : Réparation des tendons lésés de rat avec une composition lipophile de l'invention complexée à l'ADNc codant le PDGF

### A. Matériel et Méthodes

Il a été rapporté qu'un traitement avec la protéine recombinante PDGF induisait une nette amélioration de la régénération du tendon d'Achille de rat lésé. Nous avons évalué l'influence du transfert du gène codant le PDGF (PBlast45-hbFGF, InvivoGen) dans la réparation du tendon lésé en utilisant les liposomes **8a/9**. Pour cela, des analyses histologiques ont été effectuées 3 ou 6 jours après une lésion suivie ou non d'un traitement. Ce plasmide possède le gène codant le facteur de croissance FGF-2 (bFGF) (Facteur basique de croissance fibroblastique humain) sous contrôle du promoteur constitutif EF-1α-eIF4g (promoteur hybride du facteur d'élongation EF-1α humain et de la partie 5' non traduite du facteur d'initiation eIF4g).

### B. Résultats

Les résultats sont représentés sur la Figure 9.

Comparées aux coupes de tissus non lésés (Figure 9A-1, A-2 et 9B-1), les coupes de tendons lésés présentent des dégénérescences (Figure 9C-1 et B-2), Une amélioration de l'aspect tissulaire est observée sur les coupes des tissus traités au 3^{ème} et de façon plus nette au 6^{éme} jour (Figures 9D-1, D-2 et 9F-1). Par contre, sur les tendons témoins traités avec un plasmide non relevant (pNF-CMV-luc), le tissu apparaît plus désorganisé (Figure 9C-1, B-2 et E-1, C-2).

Ce vecteur ouvre donc la possibilité d'utiliser avec une bonne efficacité un gène d'intérêt thérapeutique pour la réparation de la lésion du tendon voire des pathologies liées au tendon en général. Ces pathologies sont nombreuses et courantes. Elles touchent aussi bien les sportifs, où les tendinopathies représentent 50% des blessures liées à la pratique de l'activité physique, que le reste de la population.

## Revendications

1. Utilisation d'un composé lipophile de formule (I) suivante : 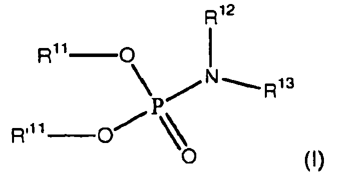
(i) R¹¹ et R'¹¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R¹² est un atome d'hydrogène ou une chaîne alkyle ayant de 1 à 4 atomes de carbone ;
(iii) R¹³ est choisi parmi :
(iii-1) un groupe de formule 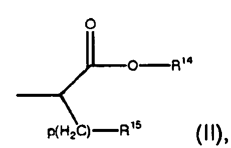 dans laquelle
- R¹⁴ est un groupe alkyle ayant de 1 à 4 atomes de carbone ;
- p est un entier égal à 1, 2, 3 ou 4 ; et
- R¹⁵ est le groupe suivant : 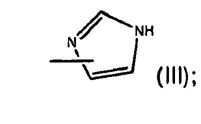 ou
(iii-2) un groupe de formule -(CH₂)_{q}-R¹⁶, dans laquelle
- q est un entier égal à 1, 2, 3 ou 4 ;
- R¹⁶ est le groupe suivant : 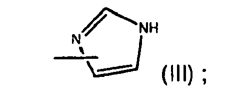 comme co-lipide pour la fabrication d'une composition de vecteur non-viral d'acide nucléique.

2. Utilisation selon la revendication 1, **caractérisée en ce que** le co-lipide de formule (I) est combiné à un composé lipophile cationique, apte à former un complexe avec un acide nucléique.

3. Composition lipophile comprenant la combinaison de deux composés lipophiles, respectivement :
a) un premier composé lipophile cationique, apte à former un complexe avec un acide nucléique ; et
et
b) un second composé lipophile de formule (I) suivante : 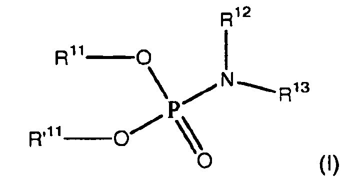
(i) R¹¹ et R'¹¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R¹² est un atome d'hydrogène ou une chaîne alkyle ayant de 1 à 4 atomes de carbone ;
(iii) R¹³ est choisi parmi :
(iii-1) un groupe de formule 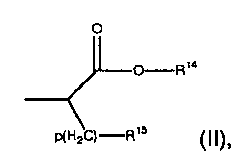 dans laquelle
- R¹⁴ est un groupe alkyle ayant de 1 à 4 atomes de carbone ;
- p est un entier égal à 1, 2, 3 ou 4 ; et
- R¹⁵ est le groupe suivant : 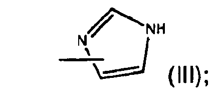 ou
(iii-2) un groupe de formule -(CH₂)_{q}-R¹⁶, dans laquelle
- q est un entier égal à 1, 2 , 3 ou 4 ;
- R¹⁶ est le groupe suivant :

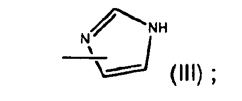

4. Composition selon la revendication 3, **caractérisée en ce que** dans le second composé lipophile, le groupe R¹⁵ est choisi parmi les groupes suivants :

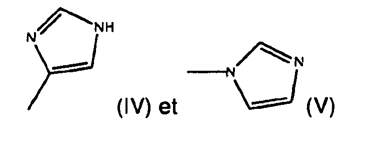

5. Composition selon la revendication 3, **caractérisée en ce que** dans le second composé lipophile, le groupe R¹⁶ est choisi parmi les groupes suivants :

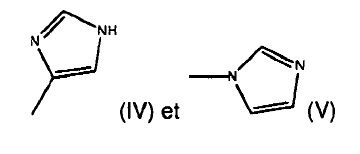

6. Composition selon la revendication 3, **caractérisée en ce que** dans le composé de formule (I), les groupes R¹¹ et R'¹¹ sont choisis parmi :
- le groupe tetradecyl,
- le groupe phytanyle
- le groupe oleyl,
- les groupes polyalcényl C_{18:2} et C_{18:3}, dans lequel le premier nombre représente le nombre d'atomes de carbone dans la chaîne alcényle et le second nombre représente le nombre de double liaisons dans la chaîne alcényle ; et
- le groupe monoalcényle C_{18 :1}, dans lequel le premier nombre représente le nombre d'atomes de carbone dans la chaîne alcényle et le second nombre représente le nombre de double liaisons dans la chaîne alcényle.

7. Composition selon la revendication 6, **caractérisée en ce que** dans le composé de formule (I), les groupes R¹¹ et R'¹¹ sont identiques.

8. Composition selon la revendication 3, **caractérisée en ce que** le premier composé lipophile cationique, apte à former un complexe avec un acide nucléique, est choisi parmi le 1,2 dioleyl-3 trimethylammonium deoxyglycerol (DOTAP), le 1,2 dioleyl-3 trimethylammonium (DOTMA), le dimethylammonium ethyloxycarbonylcholesterol (DC-chol), le bromure de dimethyldioactadecyl ammonium (DDAB), le 1,2 dimyristoyl-3 trimethylammonium deoxyglycerol, 1,2 dipalmitoyl-3 trimethylammonium deoxyglycerol, le 1,2 dioleyl-3 trimethylammonium deoxyglycerol, le 1,2 distearoyl-3 trimethylammonium deoxyglycerol, le chlorure de N-[1-[2,3-bis(oleoyloxy)]propy-1]-N,N,N-trimethylammonium, le dioctadecylamidoglycylspermine (DOGS), le trifluoroacétate de 2,3-dioleoyloxy-N-(2(sperminecarboxamido)-ethyl)-N,N-dimethyl-1-propanaminium (DOSPA), le 1,2-dioleoyl-sn-glycero-3-ethylphosphocholine (DOEPC), le 1,2-dilauroyl-sn-glycero-3-ethylphosphocholine, le 1,2-dipalmitoyl-sn-glycero-3-ethylphosphocholine, le 1,2-distearoyl-sn-glycero-3-ethylphosphocholine, le 1,2-palimitoyl-oleoyl-sn-glycero-3-ethylphosphocholine, le chlorure de 1-[2-(9(Z)-octadecenoyloxy)ethyl]-2-(8(Z)-hepadecenyl 1-3-(2-hydroxyethyl)imidazolium .(DOTIM), le chlorure de 1-[2-tetradecanoyloxy)ethyl]-2-tridecyl-3-(2-hydroxyethyl) imidazolium (DPTIM), le chlorure 1-[2-tetradecanoyloxy)ethyl]-2-tridecyl-3-(2-hydroxyethyl) imidazolium, le bromure de 1,2-dioleoyl-3-dimethyl-hydroxyethyl ammonium (DOR1); le bromure de 1,2-dioleyloxypropyl-3-dimethyl-hydroxyethyl ammonium (DORIE); le bromure de 1,2-dioleyloxypropyl-3-dimethyl-hydroxypropyl ammonium (DORIE-HP); le bromure de 1,2-dioleyloxypropyl-3-dimethyl-hydroxybutyl ammonium (DORIE-HB); le bromure de 1,2-dioleyloxypropyl-3-dimethyl-hydroxypentyl ammonium (DORIE-HPe); le bromure de 1,2-dimyristyloxypropyl-3-dimethyl-hydroxylethyl ammonium (DMRIE); le bromure de 1,2-dipalmityloxypropyl-3-dimethyl-hydroxyethyl ammonium (DPRIE), le bromure de 1,2-disteryloxypropyl-3-dimethyl-hydroxyethyl ammonium (DSRIE). ; la L-histidine-(N,N-di-n-hexadecylamine)ethylamide (lipid 1); la L-histidine-(N,N-di-n-hexadecylamine,-N-methyl)ethylamide ; L-histidine-Cholesteryl-ethylamide, alanine-cholesteryl-ethylamide, et le bis(guanidinium)-tren-cholesterol (BGTC).

9. Composition selon la revendication 3, **caractérisé en ce que** le premier composé lipophile cationique, apte à former un complexe avec un acide nucléique, est un composé lipophile de formule (XI) suivante : 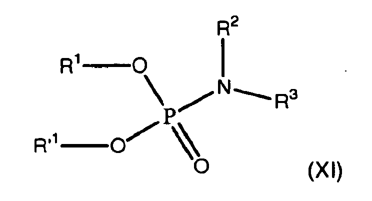 dans laquelle :
(i) R¹ et R'¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R² est une atome d'hydrogène ou une chaîne alkyle ayant de 1 à 4 atomes de carbone ;
(iii) R³ est choisi parmi :
(iii-1) un groupe de formule 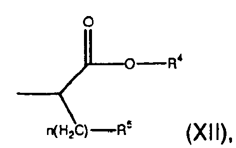 dans laquelle
- R⁴ est un groupe alkyle ayant de 1 à 4 atomes de carbone ;
- n est un entier égal à 1, 2, 3 ou 4 ; et
- R⁵ est un groupe choisi parmi : 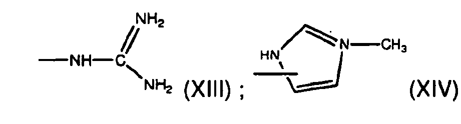 et -NH₃ ; ou
(iii-2) un groupe de formule -(CH₂)ₒ-R⁶, dans laquelle
- o est un entier égal à 1, 2, 3 ou 4 ;
- R⁶ est un groupe choisi parmi : 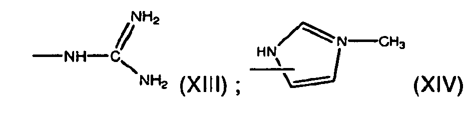 et NH₃

10. Composition selon la revendication 9, **caractérisée en ce que** dans le premier composé lipophile, le groupe R⁵ est le groupe suivant :

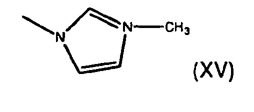

11. Composition selon la revendication 9, **caractérisée en ce que** dans le premier composé lipophile, le groupe R⁶ est le groupe suivant :

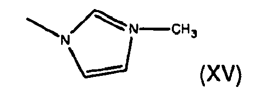

12. Composition selon la revendication 9, **caractérisée en ce que** dans le composé de formule (XI), les groupes R1 et R'1 sont choisis parmi :
- le groupe tetradecyle,
- le groupe oléyle,
- le groupe phytanyle,
- les groupes polyalcényle C_{18:2} et C_{18:3}, dans lequel le premier nombre représente le nombre d'atomes de carbone dans la chaîne alcényle et le second nombre représente le nombre de double liaisons dans la chaîne alcényle ; et
- le groupe monoalcényle C_{18 :1}, dans lequel le premier nombre représente le nombre d'atomes de carbone dans la chaîne alcényle et le second nombre représente le nombre de double liaisons dans la chaîne alcényle.

13. Composition selon la revendication 9, **caractérisée en ce que** dans le composé de formule (XI), les groupes R¹ et R^{'1} sont identiques.

14. Composition selon la revendication 9 **caractérisée en ce que** le premier composé lipophile de formule (XI) est choisi parmi :
- le dioleoyl-N- phosphoramidate de l'ester méthylique de l'arginine,
- le dioleoyl-N-phosphoramidate de l'ester méthylique de l'homoarginine,
- le dioleoyl-N- phosphoramidate de l'ester méthylique de la lysine,
- le dioleoyl-N-phosphoramidate de 2-ethylguanidinium,
- le dioleoyl-N-phosphoramidate de 4-butylguanidinium,
- le dioleoyl-N-phosphoramidate de 2-ethyl(N-methyl)imidazolium, et
- le dioleoyl-N-phosphoramidate de 3-propyl(N-methyl)imidazolium

15. Composition selon la revendication 3, **caractérisée en ce que** le second composé lipophile de formule (I) est choisi parmi :
- le dioleoyl-N- phosphoramidate de l'ester méthylique de l'histidine,
- le dioleoyl-N- phosphoramidate de 3-propylimidazole, et
- le dioleoyl-N- phosphoramidate de l'histamine

16. Composition selon la revendication 3, **caractérisée en ce qu'**elle comprend la combinaison du premier composé lipophile le dioleoyl-N-phosphoramidate de 2-ethyl(N-methyl)imidazolium et du second composé lipophile le dioleoyl-N- phosphoramidate de l'histamine,

17. Composition selon l'une des revendications 3 à 16, **caractérisée en ce qu'**elle se présente sous la forme de vésicules lipidiques.

18. Vésicule lipidique constituée essentiellement d'une composition selon l'une des revendications 3 à 17.

19. Vésicule lipidique selon la revendication 18, consistant en une vésicule unilamellaire.

20. Vésicule lipidique selon la revendication 18, consistant en une vésicule multilamellaire.

21. Utilisation d'une composition selon l'une des revendications 3 à 17 ou de vésicules lipidiques selon l'une des revendications 18 à 20, pour introduire *in vitro* un acide nucléique dans une cellule hôte.

22. Procédé pour introduire *in vitro* un acide nucléique (ADN ou ARN) dans une cellule hôte, **caractérisé en ce qu'**il comprend les étapes suivantes :
a) mettre en contact ledit acide nucléique avec une composition selon l'une des revendications 3 à 17 ou avec des vésicules lipidiques selon l'une des revendications 18 à 20 afin d'obtenir un complexe entre ledit acide nucléique, d'une part, et ladite composition ou lesdites vésicules lipidiques, d'autre part ; et
b) incuber la cellule hôte avec le complexe formé à l'étape a).

23. Procédé selon la revendication 22, **caractérisé en ce que** ladite cellule hôte est une cellule de mammifère non humain ou une cellule humaine.

24. Complexe formé entre un acide nucléique et une composition selon l'une des revendications 3 à 17, ou des vésicules lipidiques selon l'une des revendications 18 à 20.

25. Composition comprenant un complexe selon la revendication 24.

26. Composition pharmaceutique comprenant un complexe selon la revendication 24

27. Composé lipophile de formule (XI) suivante : 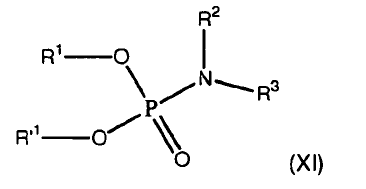 dans laquelle :
(i) R¹ et R^{'1} représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R² est une atome d'hydrogène ou une chaîne alkyle ayant de 1 à 4 atomes de carbone ;
(iii) R³ est un groupe de formule 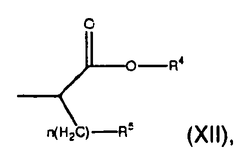 dans laquelle
- R⁴ est un groupe alkyle ayant de 1 à 4 atomes de carbone ;
- n est un entier égal à 1, 2, 3 ou 4 ; et
- R⁵ est un groupe choisi parmi : 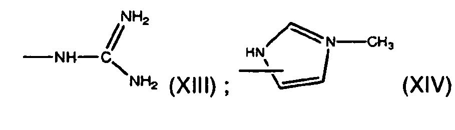 et -NH₃.

28. Composé lipophile selon la revendication 27, **caractérisé en ce que** le groupe R⁵ possède la formule suivante :

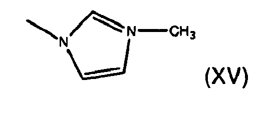

29. Composé lipophile selon l'une des revendications 27 et 28, **caractérisé en ce que** les groupes R¹ et R^{'1} sont identiques et représentent chacun la chaîne monoalcényle C_{18 :1}, dans lequel le premier nombre représente le nombre d'atomes de carbone dans la chaîne alcényle et le second nombre représente le nombre de double liaisons dans la chaîne alcényle.

30. Composé lipophile de formule (I) suivante : 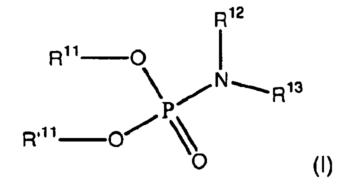
(i) R¹¹ et R'¹¹ représentent chacun, indépendamment l'un de l'autre, une chaîne alkyle de 10 à 24 atomes de carbone, une chaîne monoalcényle ou polyalcényle de 10 à 24 atomes de carbone, avec la chaîne polyalcényle ayant de 2 à 4 double liaisons, ou une chaîne monoalcynyle ou polyalcynyle de 10 à 24 atomes de carbone, avec la chaîne polyalcynyle ayant de 2 à 4 triple liaisons;
(ii) R¹² est un atome d'hydrogène ou une chaîne alkyle ayant de 1 à 4 atomes de carbone ;
(iii) R¹³ est un groupe de formule 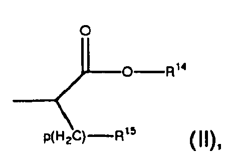 dans laquelle
- R¹⁴ est un groupe alkyle ayant de 1 à 4 atomes de carbone ;
- p est un entier égal à 1, 2, 3 ou 4 ; et
- R¹⁵ est le groupe suivant :

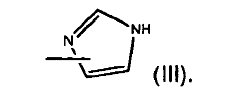

31. Composé lipophile selon la revendication 30, **caractérisé en ce que** le groupe R¹⁵ est choisi parmi les groupes suivants :

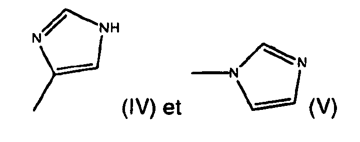

32. Composé lipophile selon la revendication 30, **caractérisé en ce que** le groupe R¹⁶ est choisi parmi les groupes suivants :

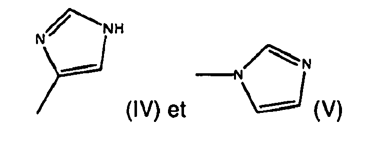

33. Composé lipophile selon la revendication 30, **caractérisé en ce que** les groupes R¹¹ et R'¹¹ sont choisis parmi :
- le groupe tetradecyl,
- le groupe oleyl, et
- les groupes polyalcényl C_{18 :2} et C_{18 :3}, dans lequel le premier nombre représente le nombre d'atomes de carbone dans la chaîne alcényle et le second nombre représente le nombre de double liaisons dans la chaîne alcényle ;
- le groupe monoalcényle C_{18 :1}, dans lequel le premier nombre représente le nombre d'atomes de carbone dans la chaîne alcényle et le second nombre représente le nombre de double liaisons dans la chaîne alcényle.

34. Composé lipophile selon la revendication 30, **caractérisé en ce que** les groupes R¹¹ et R'¹¹ sont identiques.

## Claims

1. Use of a lipophilic compound of following formula (I): 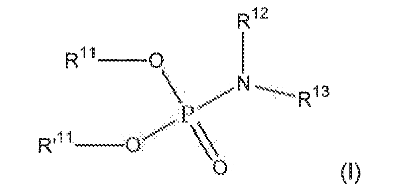 wherein
(i) R¹¹ and R'¹¹ each represent independently from each other, an alkyl chain having from 10 to 24 carbon atoms, a monoalkenyl or a polyalkenyl chain having from 10 to 24 carbon atoms, the polyalkenyl chain having from 2 to 4 double bonds, or a monoalkynyl or a polyalkynyl chain having from 10 to 24 carbon atoms, the polyalkynyl chain having from 2 to 4 triple bonds;
(ii) R¹² is a hydrogen atom or an alkyl chain having from 1 to 4 carbon atoms;
(iii) R¹³ is selected from:
(iii-1) a group of formula 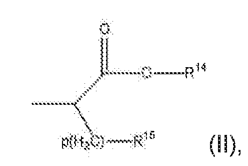 wherein
- R¹⁴ is an alkyl group having from 1 to 4 carbon atoms;
- p is an integer equal to 1, 2, 3 or 4; and
- R¹⁵ is the following group: 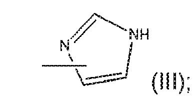 or
(iii-2) a group of formula -(CH₂)_{q}-R¹⁶, wherein
- q is an integer equal to 1, 2, 3 or 4;
- R¹⁶ is the following group: 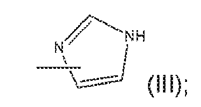 as a co-lipid for preparing a nuclei acid non viral vector composition.

2. Use according to claim 1, **characterized in that** the co-lipid of formula (I) is combined with a cationic, lipophilic compound, that is able to form a complex with a nucleic acid.

3. A lipophilic composition comprising a combination of two lipophilic compounds, respectively:
a) a first cationic, lipophilic compound, that is able to form a complex with a nucleic acid; and
b) a second lipophilic compound of following formula (I): 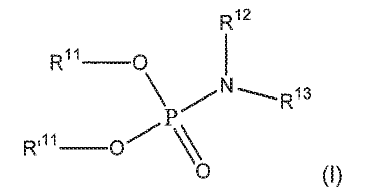 wherein
(i) R¹¹ and R'¹¹ each represent independently from each other, an alkyl chain having from 10 to 24 carbon atoms, a monoalkenyl or a polyalkenyl chain having from 10 to 24 carbon atoms, the polyalkenyl chain having from 2 to 4 double bonds, or a monoalkynyl or a polyalkynyl chain having from 10 to 24 carbon atoms, the polyalkynyl chain having from 2 to 4 triple bonds;
(ii) R¹² is a hydrogen atom or an alkyl chain having from 1 to 4 carbon atoms;
(iii) R¹³ is selected from:
(iii-1) a group of formula 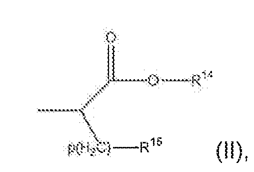 wherein
- R¹⁴ is an alkyl group having from 1 to 4 carbon atoms;
- p is an integer equal to 1, 2, 3 or 4; and
- R¹⁵ is the following group: 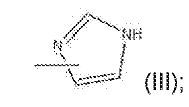 or
(iii-2) a group of formula -(CH₂)_{q}-R¹⁶, wherein
- q is an integer equal to 1, 2, 3 or 4;
- R¹⁶ is the following group

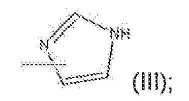

4. A composition according to claim 3, **characterized in that** in the second lipophilic compound, the R¹⁵ group is selected from the following groups:

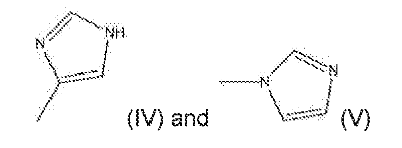

5. A composition according to claim 3, **characterized in that** in the second lipophilic compound, the R¹⁶ group is selected from the following groups:

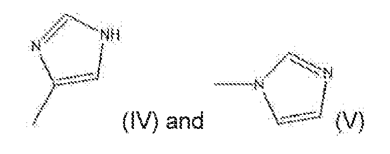

6. A composition according to claim 3, **characterized in that** in the compound of formula (I), the R¹¹ and R'¹¹ groups are selected from:
- a tetradecyl group,
- a phytanyl group
- an oleyl group,
- the polyalkenyl C_{18:2} and C_{18:3} groups, wherein the first number is the number of carbon atoms in the alkenyl chain and the second number is the number of double bonds in the alkenyl chain; and
- the C_{18:1} monoalkenyl group, wherein the first number is the number of carbon atoms in the alkenyl chain and the second number is the number of double bonds in the alkenyl chain.

7. A composition according to claim 6, **characterized in that** in the compound of formula (I), the R¹¹ and R'¹¹ groups are the same.

8. A composition according to claim 3, **characterized in that** the first cationic, lipophilic compound, that is able to form a complex with a nucleic acid, is selected from 1,2-dioleyl-3 trimethylammonium deoxyglycerol (DOTAP), 1,2-dioleyl-3 trimethylammonium (DOTMA), dimethylammonium ethyloxycarbonyl cholesterol (DC-chol), dimethyldioctadecyl ammonium bromide (DDAB), 1,2- dimyristoyl-3 trimethylammonium deoxyglycerol, 1,2-dipalmitoyl-3 trimethylammonium deoxyglycerol, 1,2-dioleyl-3 trimethylammonium deoxyglycerol, 1,2-distearoyl-3 trimethylammonium deoxyglycerol, N-[1-[2,3-bis(oleoyloxy)]propyl-1]-N,N,N-trimethylammonium chloride, dioctadecyl amidoglycylspermine (DOGS), 2,3-dioleoyloxy-N-(2(sperminecarboxamido)-ethyl)-N,N-dimethyl-1-propanaminium trifluoroacetate (DOSPA), 1,2-dioleoyl-sn-glycero-3-ethyl phosphocholine (DOEPC), 1,2-dilauroyl-sn-glycero-3-ethyl phosphocholine, 1,2-dipalmitoyl-sn-glycero-3-ethyl phosphocholine, 1,2-distearoyl-sn-glycero-3-ethyl phosphocholine, 1,2-palmitoyl-oleoyl-sn-glycero-3-ethyl phosphocholine, 1-[2-(9(Z)-octadecenoyloxy)ethyl]-2-(8(Z)-heptadecenyl 1-3-(2-hydroxyethyl)imidazolium chloride .(DOTIM), 1-[2-tetradecanoyloxy)ethyl]-2-tridecyl-3-(2-hydroxyethyl) imidazolium chloride (DPTIM), 1-[2-tetradecanoyloxy)ethyl]-2-tridecyl-3-(2-hydroxyethyl) imidazolium chloride, 1,2-dioleoyl-3-dimethyl-hydroxyethyl ammonium bromide (DOR1); 1,2-dioleyloxypropyl-3-dimethyl-hydroxyethyl ammonium bromide (DORIE); 1,2-dioleyloxypropyl-3-dimethylhydroxypropyl ammonium bromide (DORIE-HP); 1,2-dioleyloxypropyl-3-dimethylhydroxybutyl ammonium bromide (DORIE-HB); 1,2-dioleyloxypropyl-3-dimethylhydroxypentyl ammonium bromide (DORIE-HPe); 1,2-dimyristyloxypropyl-3-dimethylhydroxyethyl ammonium bromide (DMRIE); 1,2-dipalmityloxypropyl-3-dimethylhydroxyethyl ammonium bromide (DPRIE), 1,2-disteryloxypropyl-3-dimethylhydroxyethyl ammonium bromide (DSRIE).; L-histidine-(N,N-di-n-hexadecylamine)ethylamide (lipid 1); L-histidine-(N,N-di-n-hexadecylamine-N-methyl)ethylamide; L-histidine-cholesteryl-ethylamide, alanine-cholesteryl-ethylamide, and bis(guanidinium)-tren-cholesterol (BGTC),

9. A composition according to claim 3, **characterized in that** the first cationic, lipophilic compound, that is able to form a complex with a nucleic acid, is a lipophilic compound of following formula (XI): 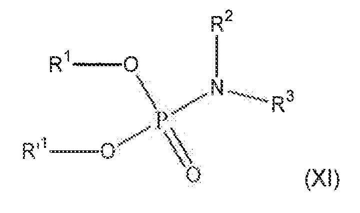 wherein:
(i) R¹ and R'¹ each represent independently from each other, an alkyl chain having from 10 to 24 carbon atoms, a monoalkenyl or a polyalkenyl chain having from 10 to 24 carbon atoms, the polyalkenyl chain having from 2 to 4 double bonds, or a monoalkynyl or a polyalkynyl chain having from 10 to 24 carbon atoms, the polyalkynyl chain having from 2 to 4 triple bonds;
(ii) R² is a hydrogen atom or an alkyl chain having from 1 to 4 carbon atoms;
(iii) R³ is selected from:
(iii-1) a group of formula 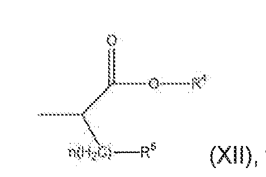 wherein
- R⁴ is an alkyl group having from 1 to 4 carbon atoms;
- n is an integer equal to 1, 2, 3 or 4; and
- R⁵ is a group selected from: 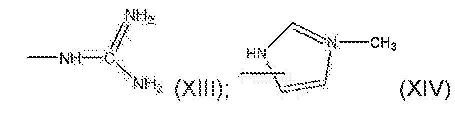 and -NH₃; or
(iii-2) a group of formula -(CH2)ₒ-R⁶, wherein
- o is an integer equal to 1, 2, 3 or 4;
- R⁶ is a group selected from: 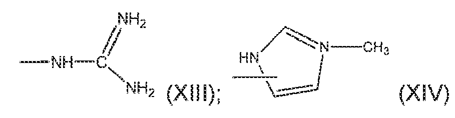 and NH₃

10. A composition according to claim 9, **characterized in that** in the first lipophilic compound, the R⁵ group is the following group:

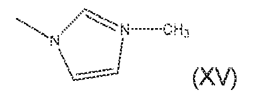

11. A composition according to claim 9, **characterized in that** in the first lipophilic compound, the R⁶ group is the following group:

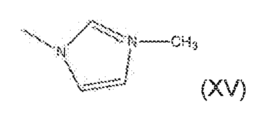

12. A composition according to claim 9, **characterized in that** in the compound of formula (XI), the R1 and R'1 groups are selected from:
- a tetradecyl group,
- an oleyl group,
- a phytanyl group,
- C_{18:2} and C_{18:3} polyalkenyl groups, wherein the first number is the number of carbon atoms in the alkenyl chain and the second number is the number of double bonds in the alkenyl chain; and
- C_{18:1} monoalkenyl group, wherein the first number is the number of carbon atoms in the alkenyl chain and the second number is the number of double bonds in the alkenyl chain.

13. A composition according to claim 9, **characterized in that** in the compound of formula (XI), the R¹ and R'¹ groups are the same.

14. A composition according to claim 9, **characterized in that** the first lipophilic compound of formula (XI) is selected from:
- arginine methyl ester dioleoyl-N- phosphoramidate,
- homoarginine methyl ester dioleoyl-N-phosphoramidate,
- lysine methyl ester dioleoyl-N- phosphoramidate,
- 2-ethylguanidinium dioleoyl-N-phosphoramidate,
- 4-butylguanidinium dioleoyl-N-phosphoramidate,
- 2-ethyl(N-methyl)imidazolium dioleoyl-N-phosphoramidate, and
- 3-propyl(N-methyl)imidazolium dioleoyl-N-phosphoramidate.

15. A composition according to claim 3, **characterized in that** the second lipophilic compound of formula (I) is selected from:
- histidine methyl ester dioleoyl-N- phosphoramidate,
- 3-propylimidazole dioleoyl-N- phosphoramidate, and
- histamine dioleoyl-N- phosphoramidate.

16. A composition according to claim 3, **characterized in that** it comprises the combination of the first lipophilic compound 2-ethyl(N-methyl)imidazolium dioleoyl-N-phosphoramidate and of the second lipophilic compound histamine dioleoyl-N-phosphoramidate.

17. A composition according to any claim 3 to 16, **characterized in that** it presents in the form of lipid vesicles,

18. A lipid vesicle substantially formed of a composition according to any claim 3 to 17.

19. A lipid vesicle according to claim 18, consisting in an unilamellar vesicle.

20. A lipid vesicle according to claim 18, consisting in a multilamellar vesicle.

21. Use of a composition according to any claim 3 to 17 or of lipid vesicles according to any claim 18 to 20, for introducing *in vitro* a nucleic acid into a host cell.

22. A method for introducing *in vitro* a nucleic acid (DNA or RNA) into a host cell, **characterized in that** it comprises the following steps of:
a) contacting said nucleic acid with a composition according to any claim 3 to 17 or with lipid vesicles according to any claim 18 to 20 so as to obtain a complex between said nucleic acid, on the one hand, and said composition or said lipid vesicles, on the other hand; and
b) incubating the host cell with the complex formed in step a).

23. A method according to claim 22, **characterized in that** said host cell is a non human mammal cell or a human cell.

24. A complex formed between a nucleic acid and a composition according to any claim 3 to 17, or lipid vesicles according to any claim 18 to 20.

25. A composition comprising a complex according to claim 24.

26. A pharmaceutical composition comprising a complex according to claim 24.

27. A lipophilic compound of following formula (XI): 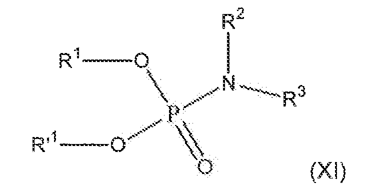 wherein:
(i) R¹ and R'¹ each represent independently from each other, an alkyl chain having from 10 to 24 carbon atoms, a monoalkenyl or a polyalkenyl chain having from 10 to 24 carbon atoms, the polyalkenyl chain having from 2 to 4 double bonds, or a monoalkynyl or a polyalkynyl chain having from 10 to 24 carbon atoms, the polyalkynyl chain having from 2 to 4 triple bonds;
(ii) R² is a hydrogen atom or an alkyl chain having from 1 to 4 carbon atoms;
(iii) R³ is a group of formula 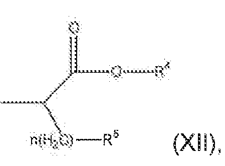 wherein
- R⁴ is an alkyl group having from 1 to 4 carbon atoms;
- n is an integer equal to 1, 2, 3 or 4; and
- R⁵ is a group selected from: 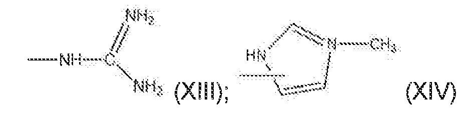 and -NH₃,

28. A lipophilic compound according to claim 27, **characterized in that** the R⁵ group has the following formula:

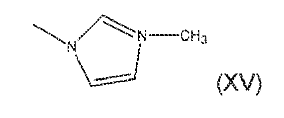

29. A lipophilic compound according to any one of claims 27 and 28, wherein the R¹ and R'¹ groups are the same and each represent C_{18:1} monoalkenyl chain, wherein the first number is the number of carbon atoms in the alkenyl chain and the second number is the number of double bonds in the alkenyl chain.

30. A lipophilic compound of following formula (I), 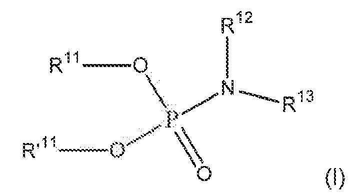 wherein
(i) R¹¹ and R'¹¹ each represent independently from each other, an alkyl chain having from 10 to 24 carbon atoms, a monoalkenyl or a polyalkenyl chain having from 10 to 24 carbon atoms, the polyalkenyl chain having from 2 to 4 double bonds, or a monoalkynyl or a polyalkynyl chain having from 10 to 24 carbon atoms, the polyalkynyl chain having from 2 to 4 triple bonds;
(ii) R¹² is a hydrogen atom or an alkyl chain having from 1 to 4 carbon atoms;
(iii) R¹³ is a group of formula 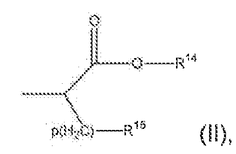 wherein
- R¹⁴ is an alkyl group having from 1 to 4 carbon atoms;
- p is an integer equal to 1, 2, 3 or 4; and
- R¹⁵ is the following group:

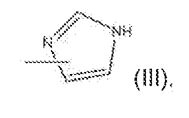

31. A lipophilic compound according to claim 30, **characterized in that** the R¹⁵ group is selected from the following groups:

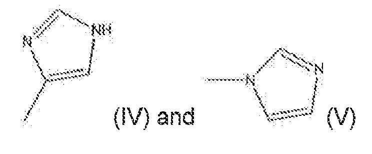

32. A lipophilic compound according to claim 30, **characterized in that** the R¹⁶ group is selected from the following groups:

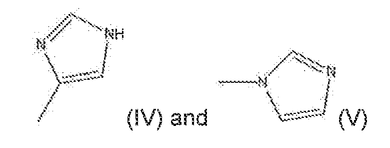

33. A lipophilic compound according to claim 30, **characterized in that** the R¹¹ and R'¹¹ groups are selected from:
- a tetradecyl group,
- an oleyl group, and
- C_{18:2} and C_{18:3} polyalkenyl groups, wherein the first number is the number of carbon atoms in the alkenyl chain and the second number is the number of double bonds in the alkenyl chain;
- C_{18:1} monoalkenyl group, wherein the first number is the number of carbon atoms in the alkenyl chain and the second number is the number of double bonds in the alkenyl chain.

34. A Lipophilic compound according to claim 30, **characterized in that** the R¹¹ and R'¹¹ groups are the same.

## Patentansprüche

1. Verwendung einer lipophilen Verbindung der folgenden Formel (I): 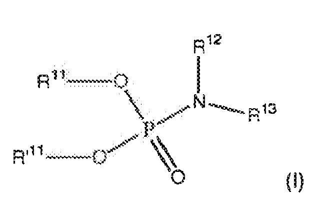 wobei
(i) R¹¹ und R'¹¹ jeweils unabhängig für eine Alkylkette mit 10 bis 24 Kohlenstoffatomen, eine Monoalkenyl- oder Polyalkenylkette mit 10 bis 24 Kohlenstoffatomen, wobei die Polyalkenylkette 2 bis 4 Doppelbindungen aufweist, oder eine Monoalkinyl- oder Polyalkinylkette mit 10 bis 24 Kohlenstoffatomen, wobei die Polyalkinylkette 2 bis 4 Dreifachbindungen aufweist, stehen;
(ii) R¹² ein Wasserstoffatom oder eine Alkylkette mit 1 bis 4 Kohlenstoffatomen ist;
(iii) R¹³ ausgewählt ist aus:
(iii-1) einer Gruppe der Formel 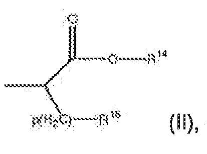 wobei
- R¹⁴ eine Alkylgruppe mit 1 bis 4 Kohlenstoffatomen ist;
- p eine ganze Zahl von 1, 2, 3 oder 4 ist; und
- R¹⁵ die folgende Gruppe ist: 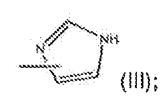 oder (iii-2) einer Gruppe der Formel -(CH₂)_{q}-R¹⁶, wobei
- q eine ganze Zahl von 1, 2, 3 oder 4 ist;
- R¹⁶ die folgende Gruppe ist: 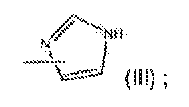
als Colipid für die Herstellung einer nichtviralen Nucleinsäure-Vektorzusammensetzung.

2. Verwendung gemäß Anspruch 1, **dadurch gekennzeichnet, dass** das Colipid der Formel (I) mit einer kationischen lipophilen Verbindung kombiniert ist, die einen Komplex mit einer Nucleinsäure bilden kann,

3. Lipophile Zusammensetzung, die die Kombination von zwei lipophilen Verbindungen umfasst, und zwar:
a) einer ersten kationischen lipophilen Verbindung, die einen Komplex mit einer Nucleinsäure bilden kann; und
b) einer zweiten lipophilen Verbindung der folgenden Formel (I); 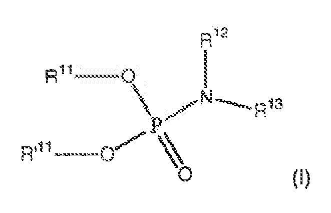 wobei
(i) R¹¹ und R'¹¹ jeweils unabhängig für eine Alkylkette mit 10 bis 24 Kohlenstoffatomen, eine Monoalkenyl- oder Polyalkenylkette mit 10 bis 24 Kohlenstoffatomen, wobei die Polyalkenylkette 2 bis 4 Doppelbindungen aufweist, oder eine Monoalkinyl- oder Polyalkinylkette mit 10 bis 24 Kohlenstoffatomen, wobei die Polyalkinylkette 2 bis 4 Dreifachbindungen aufweist, stehen;
(ii) R¹² ein Wasserstoffatom oder eine Alkylkette mit 1 bis 4 Kohlenstoffatomen ist;
(iii) R¹³ ausgewählt ist aus:
(iii-1) einer Gruppe der Formel 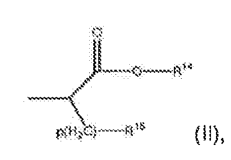 wobei
- R¹⁴ eine Alkylgruppe mit 1 bis 4 Kohlenstoffatomen ist;
- p eine ganze Zahl von 1, 2, 3 oder 4 ist; und
- R¹⁵ die folgende Gruppe ist: 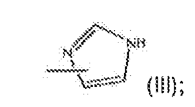 oder
(iii-2) einer Gruppe der Formel -(CH₂)_{q}-R¹⁶, wobei
- q eine ganze Zahl von 1, 2, 3 oder 4 ist;
- R¹⁶ die folgende Gruppe ist:

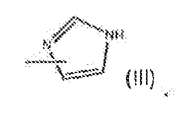

4. Zusammensetzung gemäß Anspruch 3, **dadurch gekennzeichnet, dass** die Gruppe R¹⁵ in der zweiten lipophilen Verbindung aus den folgenden Gruppen ausgewählt ist:

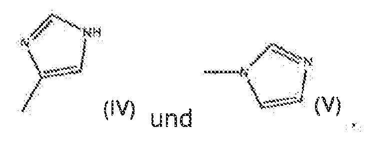

5. Zusammensetzung gemäß Anspruch 3, **dadurch gekennzeichnet, dass** die Gruppe R¹⁶ in der zweiten lipophilen Verbindung aus den folgenden Gruppen ausgewählt ist:

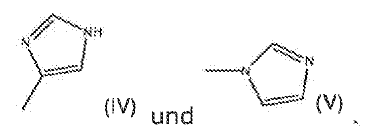

6. Zusammensetzung gemäß Anspruch 3, **dadurch gekennzeichnet, dass** die Gruppen R¹¹ und R'¹¹ in der Verbindung der Formel (I) ausgewählt sind aus:
- der Tetradecylgruppe;
- der Phytanylgruppe;
- der Oleylgruppe;
- den C_{18:2}- und C_{18:3}-Polyalkenylgruppen, wobei die erste Zahl für die Zahl der Kohlenstoffatome in der Alkenylkette steht und die zweite Zahl für die Zahl der Doppelbindungen in der Alkenylkette steht; und
- der C_{18:1}-Monoalkenylgruppe, wobei die erste Zahl für die Zahl der Kohlenstoffatome in der Alkenylkette steht und die zweite Zahl für die Zahl der Doppelbindungen in der Alkenylkette steht.

7. Zusammensetzung gemäß Anspruch 6, **dadurch gekennzeichnet, dass** die Gruppen R¹¹ und R'¹¹ in der Verbindung der Formel (I) gleich sind.

8. Zusammensetzung gemäß Anspruch 3, **dadurch gekennzeichnet, dass** die erste kationische lipophile Verbindung, die einen Komplex mit einer Nucleinsäure bilden kann, ausgewählt ist aus 1,2-Dioleyl-3-trimethylammoniumdesoxyglycerin (DOTAP), 1,2-Dioleyl-3-trimethylammonium (DOTMA), Dimethylammoniumethyloxycarbonylcholesterin (DC-chol), Dimethyldioctadecylammoniumbromid (DDAB), 1,2-Dimyristoyl-3-trimethylammoniumdesoxyglycerin, 1,2-Dipalmitoyl-3-trimethylammoniumdesoxyglycerin, 1,2-Dioleyl-3-trimethylammoniumdesoxyglycerin, 1,2-distearyl-3-trimethylammoniumdesoxyglycerin, N-[1-[2,3-Bis(oleoyloxy)]propyl]-N,N,N-trimethylammoniumchlorid, Dioctadecylamidoglycylspermin (DOGS), 2,3-Dioleoyloxy-N-(2-(sperimincarboxamido)ethyl)-N,N-dimethyl-1-propanaminiumtrifluoracetat (DOSPA), 1,2-Dioleoyl-sn-glycero-3-ethylphosphocholin (DOEPC), 1,2-Dilauroyl-sn-glycero-3-ethylphosphocholin, 1,2-Dipalmitoyl-sn-glycero-3-ethylphosphocholin, 1,2-Distearoyl-sn-glycero-3-ethylphosphocholin, 1,2-Palmitoyloleoyl-sn-glycero-3-ethylphosphocholin, 1-[2-(9(Z)-Octadecenoyloxy)ethyl]-2-(8(Z)-heptadecenyl-1-3-(2-hydroxyethyl)imidazoliumchlorid (DOTIM), 1-[2-Tetradecanoyloxy)ethyl]-2-tridecyl-3-(2-hydroxyethyl)imidazoliumchlorid (DPTIM), 1-[2-Tetradecanoyloxy)ethyl]-2-tridecyl-3-(2-hydroxyethyl)imidazoliumchlorid, 1,2-Dioleoyl-3-dimethylhydroxyethylammoniumbromid (DOR1), 1,2-Dioleyloxypropyl-3-dimethylhydroxyethylammoniumbromid (DORIE), 1,2-Dioleyloxypropyl-3-dimethylhydroxypropylammoniumbromid (DORIE-HP); 1,2-Dioleyloxypropyl-3-dimethylhydroxybutylammoniumbromid (DORIE-HB); 1,2-Dioleyloxypropyl-3-dimethylhydroxypentylammoniumbromid (DORIE-HPe); 1,2-Dimyristyloxypropyl-3-dimethylhydroxylethylammoniumbromid (DMRIE); 1,2-Dipalmityloxypropyl-3-dimethylhydroxyethylammoniumbromid (DPRIE), 1,2-Disteryloxypropyl-3-dimethylhydroxyethylammoniumbromid (DSRIE), L-Histidin-(N,N-di-n-hexadecylamin)ethylamid (Lipid 1), L-Histidin-(N,N-di-n-hexadecylamin-N-methyl)ethylamid, L-Histidincholesterylethylamid, Alanincholesterylethylamid und Bis(guanidinium)-tren-cholesterin (BGTC).

9. Zusammensetzung gemäß Anspruch 3, **dadurch gekennzeichnet, dass** die erste kationische lipophile Verbindung, die einen Komplex mit einer Nucleinsäure bilden kann, eine lipophile Verbindung der folgenden Formel (XI) ist: 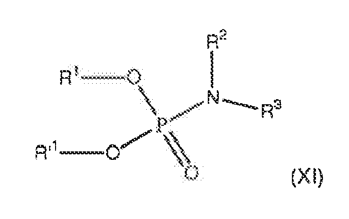 wobei:
(i) R¹ und R'¹ jeweils unabhängig für eine Alkylkette mit 10 bis 24 Kohlenstoffatomen, eine Monoalkenyl- oder Polyalkenylkette mit 10 bis 24 Kohlenstoffatomen, wobei die Polyalkenylkette 2 bis 4 Doppelbindungen aufweist, oder eine Monoalkinyl- oder Polyalkinylkette mit 10 bis 24 Kohlenstoffatomen, wobei die Polyalkinylkette 2 bis 4 Dreifachbindungen aufweist, stehen;
(ii) R² ein Wasserstoffatom oder eine Alkylkette mit 1 bis 4 Kohlenstoffatomen ist;
(iii) R³ ausgewählt ist aus:
(iii-1) einer Gruppe der Formel 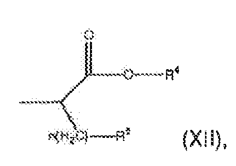 wobei
- R⁴ eine Alkylgruppe mit 1 bis 4 Kohlenstoffatomen ist;
- n eine ganze Zahl von 1, 2, 3 oder 4 ist; und
- R⁵ eine Gruppe ist, die ausgewählt ist aus: 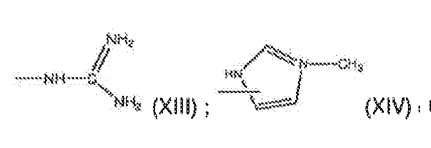 und -NH₃; oder (iii-2) einer Gruppe der Formel -(CH₂)ₒ-R⁶, wobei
- o eine ganze Zahl von 1, 2, 3 oder 4 ist;
- R⁶ eine Gruppe ist, die ausgewählt ist aus: 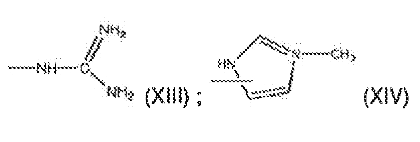 und NH₃.

10. Zusammensetzung gemäß Anspruch 9, **dadurch gekennzeichnet, dass** die Gruppe R⁵ in der ersten lipophilen Verbindung die folgenden Gruppe ist:

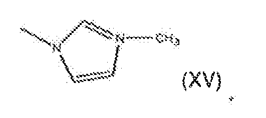

11. Zusammensetzung gemäß Anspruch 9, **dadurch gekennzeichnet, dass** die Gruppe R⁶ in der ersten lipophilen Verbindung die folgenden Gruppe ist:

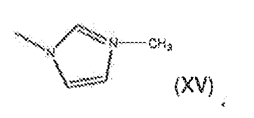

12. Zusammensetzung gemäß Anspruch 9, **dadurch gekennzeichnet, dass** die Gruppen R¹ und R'¹ in der Verbindung der Formel (XI) ausgewählt sind aus:
- der Tetradecylgruppe;
- der Oleylgruppe;
- der Phytanylgruppe;
- den C_{18:2}- und C_{18:3}-Polyalkenylgruppen, wobei die erste Zahl für die Zahl der Kohlenstoffatome in der Alkenylkette steht und die zweite Zahl für die Zahl der Doppelbindungen in der Alkenylkette steht; und
- der C_{18:1}-Monoalkenylgruppe, wobei die erste Zahl für die Zahl der Kohlenstoffatome in der Alkenylkette steht und die zweite Zahl für die Zahl der Doppelbindungen in der Alkenylkette steht.

13. Zusammensetzung gemäß Anspruch 9, **dadurch gekennzeichnet, dass** die Gruppen R¹ und R'¹ in der Verbindung der Formel (XI) gleich sind.

14. Zusammensetzung gemäß Anspruch 9, **dadurch gekennzeichnet, dass** die erste lipophile Verbindung der Formel (XI) ausgewählt ist aus:
- dem Dioleoyl-N-phosphoramidat des Argininmethylesters,
- dem Dioleoyl-N-phosphoramidat des Homoargininmethylesters,
- dem Dioleoyl-N-phosphoramidat des Lysinmethylesters,
- 2-Ethylguanidiniumdioleoyl-N-phosphoramidat,
- 4-Butylguanidiniumdioleoyl-N-phosphoramidat,
- 2-Ethyl(N-methyl)imidazoliumdioleoyl-N-phosphoramidat und
- 3-Propyl(N-methyl)imidazoliumdioleoyl-N-phosphoramidat.

15. Zusammensetzung gemäß Anspruch 3, **dadurch gekennzeichnet, dass** die zweite lipophile Verbindung der Formel (XI) ausgewählt ist aus:
- dem Dioleoyl-N-phosphoramidat des Histidinmethylesters,
- dem Dioleoyl-N-phosphoramidat von 3-Propylimidazol und
- dem Dioleoyl-N-phosphoramidat von Histamin.

16. Zusammensetzung gemäß Anspruch 3, **dadurch gekennzeichnet, dass** sie die Kombination der ersten lipophilen Verbindung, 2-Ethyl(N-methyl)-imidazoliumdioleoyl-N-phosphoramidat, und der zweiten lipophilen Verbindung, dem Dioleoyl-N-phosphoramidat von Histamin, umfasst.

17. Zusammensetzung gemäß einem der Ansprüche 3 bis 16, **dadurch gekennzeichnet, dass** sie in Form von Upidvesikeln vorliegt.

18. Lipidvesikel, das im Wesentlichen aus einer Zusammensetzung gemäß einem der Ansprüche 3 bis 17 besteht.

19. Lipidvesikel gemäß Anspruch 18, das aus einem unilamellaren Vesikel besteht.

20. Upidvesikel gemäß Anspruch 18, das aus einem multilamellaren Vesikel besteht.

21. Verwendung einer Zusammensetzung gemäß einem der Ansprüche 3 bis 17 oder von Lipidvesikeln gemäß einem der Ansprüche 18 bis 20 zur in-vitro-Einführung einer Nucleinsäure in eine Wirtszelle.

22. Verfahren zur in-vitro-Einführung einer Nucleinsäure (DNA oder RNA) in eine Wirtszelle, **dadurch gekennzeichnet, dass** es die folgenden Schritte umfasst:
a) In-Kontakt-Bringen der Nucleinsäure mit einer Zusammensetzung gemäß einem der Ansprüche 3 bis 17 oder mit Lipidvesikeln gemäß einem der Ansprüche 18 bis 20, wobei man einen Komplex zwischen der Nucleinsäure einerseits und der Zusammensetzung oder den Lipidvesikeln andererseits erhält; und
b) Inkubieren der Wirtszelle mit dem in Schritt a) gebildeten Komplex.

23. Verfahren gemäß Anspruch 22, **dadurch gekennzeichnet, dass** die Wirtszelle eine nichthumane Säugerzelle oder eine humane Zelle ist.

24. Komplex, der zwischen einer Nucleinsäure und einer Zusammensetzung gemäß einem der Ansprüche 3 bis 17 oder Lipidvesikeln gemäß einem der Ansprüche 18 bis 20 gebildet ist.

25. Zusammensetzung, die einen Komplex gemäß Anspruch 24 umfasst,

26. Pharmazeutische Zusammensetzung, die einen Komplex gemäß Anspruch 24 umfasst.

27. Lipophile Verbindung der folgenden Formel (XI): 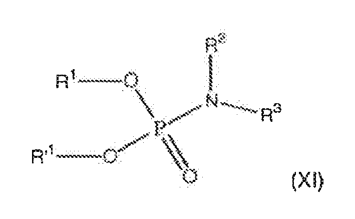 wobei:
(i) R¹ und R'¹ jeweils unabhängig für eine Alkylkette mit 10 bis 24 Kohlenstoffatomen, eine Monoalkenyl- oder Polyalkenylkette mit 10 bis 24 Kohlenstoffatomen, wobei die Polyalkenylkette 2 bis 4 Doppelbindungen aufweist, oder eine Monoalkinyl- oder Polyalkinylkette mit 10 bis 24 Kohlenstoffatomen, wobei die Polyalkinylkette 2 bis 4 Dreifachbindungen aufweist, stehen;
(ii) R² ein Wasserstoffatom oder eine Alkylkette mit 1 bis 4 Kohlenstoffatomen ist;
(iii) R³ eine Gruppe der Formel 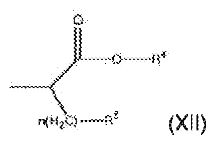 ist, wobei
- R⁴ eine Alkylgruppe mit 1 bis 4 Kohlenstoffatomen ist;
- n eine ganze Zahl von 1, 2, 3 oder 4 ist; und
- R⁵ eine Gruppe ist, die ausgewählt ist aus: 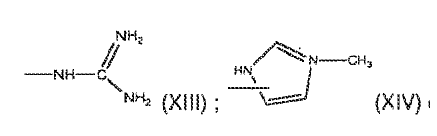 und -NH₃.

28. Lipophile Verbindung gemäß Anspruch 27, **dadurch gekennzeichnet, dass** die Gruppe R⁵ die folgende Formel besitzt:

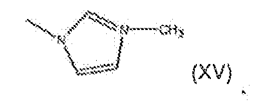

29. Lipophile Verbindung gemäß einem der Ansprüche 27 und 28, **dadurch gekennzeichnet, dass** die Gruppen R¹ und R'¹ gleich sind und jeweils für die C_{18:1}-Monoalkenylkette stehen, wobei die erste Zahl für die Zahl der Kohlenstoffatome in der Alkenylkette steht und die zweite Zahl für die Zahl der Doppelbindungen in der Alkenylkette steht.

30. Lipophile Verbindung der folgenden Formel (I): 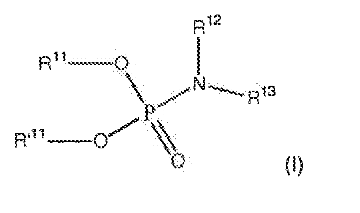 wobei
(i) R¹¹ und R'¹¹ jeweils unabhängig für eine Alkylkette mit 10 bis 24 Kohlenstoffatomen, eine Monoalkenyl- oder Polyalkenylkette mit 10 bis 24 Kohlenstoffatomen, wobei die Polyalkenylkette 2 bis 4 Doppelbindungen aufweist, oder eine Monoalkinyl- oder Polyalkinylkette mit 10 bis 24 Kohlenstoffatomen, wobei die Polyalkinylkette 2 bis 4 Dreifachbindungen aufweist, stehen;
(ii) R¹² ein Wasserstoffatom oder eine Alkylkette mit 1 bis 4 Kohlenstoffatomen ist;
(iii) R¹³ eine Gruppe der Formel 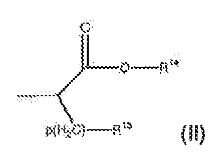 ist, wobei
- R¹⁴ eine Alkylgruppe mit 1 bis 4 Kohlenstoffatomen ist;
- p eine ganze Zahl von 1, 2, 3 oder 4 ist; und
- R¹⁵ die folgende Gruppe ist:

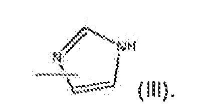

31. Lipophile Verbindung gemäß Anspruch 30, **dadurch gekennzeichnet, dass**
die Gruppe R¹⁵ aus den folgenden Gruppen ausgewählt ist ;

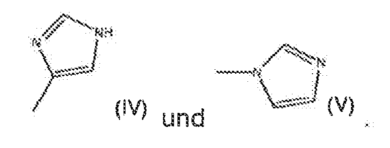

32. Lipophile Verbindung gemäß Anspruch 30, **dadurch gekennzeichnet, dass** die Gruppe R¹⁶ aus den folgenden Gruppen ausgewählt ist :

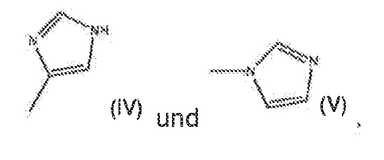

33. Lipophile Verbindung gemäß Anspruch 30, **dadurch gekennzeichnet, dass** die Gruppen R¹¹ und R'¹¹ ausgewählt sind aus:
- der Tetradecylgruppe;
- der Oleylgruppe; und
- den C_{18:2}- und C_{18:3}-Polyalkenylgruppen, wobei die erste Zahl für die Zahl der Kohlenstoffatome in der Alkenylkette steht und die zweite Zahl für die Zahl der Doppelbindungen in der Alkenylkette steht; und
- der C_{18:1}-Monoalkenylgruppe, wobei die erste Zahl für die Zahl der Kohlenstoffatome in der Alkenylkette steht und die zweite Zahl für die Zahl der Doppelbindungen in der Alkenylkette steht.

34. Lipophile Verbindung gemäß Anspruch 30, **dadurch gekennzeichnet, dass** die Gruppen R¹¹ und R'¹¹ gleich sind.
